# EUROPEAN PATENT APPLICATION

(11) **EP 3 521 439 A1**
(43) Date of publication of application: **07.08.2019**
(21) Application number: 18305100.2
(22) Date of filing: 31.01.2018
(51) Int. Cl.: C12P 7/64, C12N 9/02

(54) **METHOD FOR THE PRODUCTION OF TRIACYLGLYCERIDES AND FATTY ACIDS**

(71) Applicant: Commissariat à l'Energie Atomique et aux Energies Alternatives, 75015 Paris (FR)
(72) Inventor: Amato, Alberto, 38000 Grenoble (FR); Dellero, Younes, 38000 Grenoble (FR); Jouhet, Juliette, 38170 Seyssinet (FR); Maréchal, Eric, 38000 Grenoble (FR); Rebeille, Fabrice, 38340 Voreppe (FR)
(74) Representative: Gevers & Orès

(57) **Abstract**

The invention pertains to a method for the production of triacylglycerides (TAGs or Triacylglyerols) and fatty acids by the recombinant expression of a Δ11 fatty acid desaturase in protists.

SEQUENCE LISTING

<110>  COMMISSARIAT A L'ENERGIE ATOMIQUE ET AUX ENERGIES
       ALTERNATIVES

<120>  METHOD FOR THE PRODUCTION OF TRIACYLGLYCERIDES AND FATTY ACIDS

<130>  F0263S00657/OEB/EP

<160>  17

<170>  PatentIn version 3.5

<210>  1
<211>  347
<212>  PRT
<213>  Thaumetopoea pityocampa

<400>  1

Met Ala Pro Asn Thr Arg Glu Asn Glu Thr Ile Tyr Asp Glu Val Glu
1               5                   10                  15

His Lys Leu Glu Lys Leu Val Pro Pro Gln Ala Gly Pro Trp Asn Tyr
            20                  25                  30

Lys Ile Val Tyr Leu Asn Leu Leu Thr Phe Ser Tyr Trp Leu Ile Ala
        35                  40                  45

Gly Ala Tyr Gly Leu Tyr Leu Cys Phe Thr Ser Ala Lys Trp Ala Thr
    50                  55                  60

Ile Ile Phe Glu Phe Ile Leu Phe Phe Phe Ala Glu Met Gly Ile Thr
65                  70                  75                  80

Ala Gly Ala His Arg Leu Trp Thr His Lys Ser Tyr Lys Ala Lys Leu
                85                  90                  95

Pro Leu Glu Ile Phe Leu Met Val Leu Asn Ser Val Ala Phe Gln Asn
            100                 105                 110

Thr Ala Thr Asp Trp Val Arg Asp His Arg Leu His His Lys Tyr Ser
        115                 120                 125

Asp Thr Asp Ala Asp Pro His Asn Ala Ala Arg Gly Leu Phe Phe Ser
    130                 135                 140

His Val Gly Trp Leu Leu Val Arg Lys His Asp Glu Val Lys Lys Arg
145                 150                 155                 160

Gly Lys Phe Thr Asp Met Ser Asp Ile Tyr Asn Asn Pro Val Leu Lys
                165                 170                 175

Phe Gln Lys Lys Tyr Ala Ile Pro Phe Ile Gly Ala Val Cys Phe Ile
            180                 185                 190

Leu Pro Thr Val Ile Pro Met Tyr Phe Trp Gly Glu Ser Leu Asn Asn
        195                 200                 205

Ala Trp His Ile Cys Ile Leu Arg Tyr Ala Met Asn Leu Asn Val Thr
    210                 215                 220

Phe Ser Val Asn Ser Leu Ala His Ile Trp Gly Asn Lys Pro Tyr Asp
225                 230                 235                 240

Lys Asp Ile Lys Pro Ala Gln Asn Phe Gly Val Thr Leu Ala Thr Phe
                245                 250                 255

Gly Glu Gly Phe His Asn Tyr His His Val Phe Pro Trp Asp Tyr Arg
            260                 265                 270

Thr Ser Glu Leu Gly Asp Asn Lys Phe Asn Phe Thr Thr Lys Phe Ile
        275                 280                 285

Asn Phe Phe Glu Arg Ile Gly Leu Ala Tyr Asp Leu Lys Thr Val Ser
    290                 295                 300

Asp Asp Val Ile Ala Gln Arg Ala Lys Arg Thr Gly Asp Gly Thr His
305                 310                 315                 320

Leu Trp Asp Cys Ala Asp Lys Asn Asn Asn Asp Val Val Gln Thr Lys
                325                 330                 335

Ala Gln Ile Asp Thr Leu Cys Thr Lys His Glu
            340                 345

<210>  2
<211>  338
<212>  PRT
<213>  Helicoverpa zea

<400>  2

Met Ala Gln Ser Tyr Gln Ser Thr Thr Val Leu Ser Glu Glu Lys Glu
1               5                   10                  15

Leu Thr Leu Gln His Leu Val Pro Gln Ala Ser Pro Arg Lys Tyr Gln
            20                  25                  30

Ile Val Tyr Pro Asn Leu Ile Thr Phe Gly Tyr Trp His Ile Ala Gly
        35                  40                  45

Leu Tyr Gly Leu Tyr Leu Cys Phe Thr Ser Ala Lys Trp Ala Thr Ile
    50                  55                  60

Leu Phe Ser Tyr Ile Leu Phe Val Leu Ala Glu Ile Gly Ile Thr Ala
65                  70                  75                  80

Gly Ala His Arg Leu Trp Ala His Lys Thr Tyr Lys Ala Lys Leu Pro
                85                  90                  95

Leu Glu Ile Leu Leu Met Val Phe Asn Ser Ile Ala Phe Gln Asn Ser
            100                 105                 110

Ala Ile Asp Trp Val Arg Asp His Arg Leu His His Lys Tyr Ser Asp
        115                 120                 125

Thr Asp Ala Asp Pro His Asn Ala Ser Arg Gly Phe Phe Tyr Ser His
    130                 135                 140

Val Gly Trp Leu Leu Val Arg Lys His Pro Glu Val Lys Lys Arg Gly
145                 150                 155                 160

Lys Glu Leu Asn Met Ser Asp Ile Tyr Asn Asn Pro Val Leu Arg Phe
                165                 170                 175

Gln Lys Lys Tyr Ala Ile Pro Phe Ile Gly Ala Val Cys Phe Ala Leu
            180                 185                 190

Pro Thr Met Ile Pro Val Tyr Phe Trp Gly Glu Thr Trp Ser Asn Ala
        195                 200                 205

Trp His Ile Thr Met Leu Arg Tyr Ile Met Asn Leu Asn Val Thr Phe
    210                 215                 220

Leu Val Asn Ser Ala Ala His Ile Trp Gly Asn Lys Pro Tyr Asp Ala
225                 230                 235                 240

Lys Ile Leu Pro Ala Gln Asn Val Ala Val Ser Val Ala Thr Gly Gly
                245                 250                 255

Glu Gly Phe His Asn Tyr His His Val Phe Pro Trp Asp Tyr Arg Ala
            260                 265                 270

Ala Glu Leu Gly Asn Asn Ser Leu Asn Leu Thr Thr Lys Phe Ile Asp
        275                 280                 285

Leu Phe Ala Ala Ile Gly Trp Ala Tyr Asp Leu Lys Thr Val Ser Glu
    290                 295                 300

Asp Met Ile Lys Gln Arg Ile Lys Arg Thr Gly Asp Gly Thr Asp Leu
305                 310                 315                 320

Trp Gly His Glu Gln Asn Cys Asp Glu Val Trp Asp Val Lys Asp Lys
                325                 330                 335

Ser Ser

<210>  3
<211>  338
<212>  PRT
<213>  Spodoptera littoralis

<400>  3

Met Ala Gln Cys Val Gln Thr Thr Thr Ile Leu Glu Gln Lys Glu Glu
1               5                   10                  15

Lys Thr Val Thr Leu Leu Val Pro Gln Ala Gly Lys Arg Lys Phe Glu
            20                  25                  30

Ile Val Tyr Phe Asn Ile Ile Thr Phe Ala Tyr Trp His Ile Ala Gly
        35                  40                  45

Leu Tyr Gly Leu Tyr Leu Cys Phe Thr Ser Thr Lys Trp Ala Thr Val
    50                  55                  60

Leu Phe Ser Phe Phe Leu Phe Val Val Ala Glu Val Gly Val Thr Ala
65                  70                  75                  80

Gly Ser His Arg Leu Trp Ser His Lys Thr Tyr Lys Ala Lys Leu Pro
                85                  90                  95

Leu Gln Ile Leu Leu Met Val Met Asn Ser Leu Ala Phe Gln Asn Thr
            100                 105                 110

Val Ile Asp Trp Val Arg Asp His Arg Leu His His Lys Tyr Ser Asp
        115                 120                 125

Thr Asp Ala Asp Pro His Asn Ala Ser Arg Gly Phe Phe Tyr Ser His
    130                 135                 140

Val Gly Trp Leu Leu Val Arg Lys His Pro Asp Val Lys Lys Arg Gly
145                 150                 155                 160

Lys Glu Ile Asp Ile Ser Asp Ile Tyr Asn Asn Pro Val Leu Arg Phe
                165                 170                 175

Gln Lys Lys Tyr Ala Ile Pro Phe Ile Gly Ala Val Cys Phe Val Leu
            180                 185                 190

Pro Thr Leu Ile Pro Val Tyr Gly Trp Gly Glu Thr Trp Thr Asn Ala
        195                 200                 205

Trp His Val Ala Met Leu Arg Tyr Ile Met Asn Leu Asn Val Thr Phe
    210                 215                 220

Leu Val Asn Ser Ala Ala His Ile Tyr Gly Lys Arg Pro Tyr Asp Lys
225                 230                 235                 240

Lys Ile Leu Pro Ser Gln Asn Ile Ala Val Ser Ile Ala Thr Phe Gly
                245                 250                 255

Glu Gly Phe His Asn Tyr His His Val Phe Pro Trp Asp Tyr Arg Ala
            260                 265                 270

Ala Glu Leu Gly Asn Asn Ser Leu Asn Phe Pro Thr Lys Phe Ile Asp
        275                 280                 285

Phe Phe Ala Trp Ile Gly Trp Ala Tyr Asp Leu Lys Thr Val Ser Lys
    290                 295                 300

Glu Met Ile Lys Gln Arg Ser Lys Arg Thr Gly Asp Gly Thr Asn Leu
305                 310                 315                 320

Trp Gly Leu Glu Asp Val Asp Thr Pro Glu Asp Leu Lys Asn Thr Lys
                325                 330                 335

Gly Glu

<210> 4
<211> 6
<212> PRT
<213> Artificial sequence

<220>
<223> Consensus sequence of His box

<220>
<221> MISC_FEATURE
<222> (5)..(5)
<223> X = T or A or S

<400> 4

His Arg Leu Trp Xaa His

1               5


<210>  5
<211>  7
<212>  PRT
<213>  Artificial sequence

<220>
<223>  Consensus sequence of His box


<220>
<221>  MISC_FEATURE
<222>  (1)..(1)
<223>  X = D or E

<220>
<221>  MISC_FEATURE
<222>  (4)..(4)
<223>  X = L or M or F or S

<220>
<221>  MISC_FEATURE
<222>  (7)..(7)
<223>  X = K or R

<400>  5

Xaa His Arg Xaa His His Xaa
1               5


<210>  6
<211>  7
<212>  PRT
<213>  Artificial sequence

<220>
<223>  Consensus sequence of His box


<220>
<221>  MISC_FEATURE
<222>  (1)..(1)
<223>  X = F or S

<220>
<221>  MISC_FEATURE
<222>  (7)..(7)
<223>  X = V or T

<400>  6

Xaa His Asn Tyr His His Xaa
1               5


<210>  7
<211>  1295
<212>  DNA
<213>  Artificial sequence

<220>
<223>  Optimized sequence for the expression of a delta11 desaturase in
      Aurantiochytrium

<400>  7
atggcgccca acacccgcga gaacgagacc atctacgatg aggttgagca taagctcgag      60

aaactcgtgc ctcctcaagc gggcccctgg aactacaaaa tcgtttatct caaccttctc     120

accttctctt actggcttat cgccggcgcc tacggtctct atctctgttt tacctccgca     180

aaatgggcca ccatcatctt cgagttcatc ctcttctttt tcgccgagat gggcatcacc     240

gcaggtgctc accgcctctg gacccataaa tcttacaaag ccaagctccc tctcgagatc     300

ttcctcatgg tgctcaattc tgttgcgttc caaaacacgg ccacggactg ggtgcgcgat     360

catcgcctcc accataagta ctccgacacc gacgcggatc ctcacaacgc tgcgcgcggt     420

ctcttcttct cccatgtcgg ttggctcctc gtccgcaagc acgacgaggt caagaagcgc     480

ggtaagttta ccgatatgtc cgatatctac aacaatcccg tgctcaagtt ccagaagaaa     540

tatgccatcc ccttcatcgg tgccgtttgc tttatcttac ctaccgtgat ccccatgtac     600

ttttggggtg agtccctcaa caacgcctgg cacatctgta tcctccgcta tgcgatgaac     660

ctcaacgtca ccttctccgt gaactccctc gcgcatatct ggggtaataa gccctacgac     720

aaggatatca aacccgctca gaacttcggt gttaccctcg cgaccttcgg tgagggtttt     780

cacaactatc accacgtgtt cccctgggac tatcgcacct ccgagctcgg cgacaacaag     840

ttcaatttca ccaccaaatt catcaatttc tttgagcgca tcggtctcgc gtatgatctc     900

aagaccgttt ccgatgacgt tatcgcgcaa cgcgccaaac gcaccggtga tggtacccat     960

ctctgggatt gcgccgataa gaataataac gatgttgttc aaaccaaagc gcaaatcgat    1020

accctctgca ccaaacatga gtacccctac gacgtgcccg actacgccta acatatgcca    1080

tggtgtcaaa accggggtta gtgacattga cttgttgaca aaaatctgta tagctagaaa    1140

actctaagca acgcttttct ttgttttatt ttttatgttt aaactccttc agaattgtag    1200

gatatcttgt tttgaaaaat ccaggactga gtttcgttgc cccatttgct tgttctcgtt    1260

tgaaatgtcg aacaatagaa atgcttgcag aatga                               1295


<210>  8
<211>  2621
<212>  DNA
<213>  Artificial sequence

<220>
<223>  Recombinant cassette for the expression of the delta11 desaturase

<400>  8
ctgcaggtag gtaggtggca gtagcgttac gaggaggagt cccgagaggg agtcggagag      60

tagaaaactg gaagtcggcg aaacaaaagg cgcagagatt tgccggaatg gagagttatc     120

gtgagactct ctgagtagac ccaagtgtcc tgtgaggcac tcgtgatagg gagggggcac 180

gggctgaagg gggctacagt aaggagagag tggcgtcagt ggggttttgc cgagaactct 240

tcgagaaaga ggaagagagg aaccgagagc gccgttgaag aggggaaaaa gcagacggtt 300

taattataat taattaagta attaattact tacttattga ttgattgatt tgagaagaga 360

agcaaagaga gagttgaaga aatagtaacg aagaatagga gaagaaaggg gcaagaaaag 420

aaaaagaaag aggagaatat tagtcgatga gcgagaacgt gcaaatccaa aacagcaaaa 480

ctcaaactca aactcaaact acaagaagcg tggcgttgca gaggcaacag ctcgaaagca 540

acacagaaca aacaaacaca ggagaggcag taaggtcaat ttcgcggccg cgctagcatg 600

gcgcccaaca cccgcgagaa cgagaccatc tacgatgagg ttgagcataa gctcgagaaa 660

ctcgtgcctc ctcaagcggg cccctggaac tacaaaatcg tttatctcaa ccttctcacc 720

ttctcttact ggcttatcgc cggcgcctac ggtctctatc tctgttttac ctccgcaaaa 780

tgggccacca tcatcttcga gttcatcctc ttctttttcg ccgagatggg catcaccgca 840

ggtgctcacc gcctctggac ccataaatct tacaaagcca agctccctct cgagatcttc 900

ctcatggtgc tcaattctgt tgcgttccaa aacacggcca cggactgggt gcgcgatcat 960

cgcctccacc ataagtactc cgacaccgac gcggatcctc acaacgctgc gcgcggtctc 1020

ttcttctccc atgtcggttg gctcctcgtc cgcaagcacg acgaggtcaa gaagcgcggt 1080

aagtttaccg atatgtccga tatctacaac aatcccgtgc tcaagttcca gaagaaatat 1140

gccatcccct tcatcggtgc cgtttgcttt atcttaccta ccgtgatccc catgtacttt 1200

tggggtgagt ccctcaacaa cgcctggcac atctgtatcc tccgctatgc gatgaacctc 1260

aacgtcacct tctccgtgaa ctccctcgcg catatctggg gtaataagcc ctacgacaag 1320

gatatcaaac ccgctcagaa cttcggtgtt accctcgcga ccttcggtga gggttttcac 1380

aactatcacc acgtgttccc ctgggactat cgcacctccg agctcggcga caacaagttc 1440

aatttcacca ccaaattcat caatttcttt gagcgcatcg gtctcgcgta tgatctcaag 1500

accgtttccg atgacgttat cgcgcaacgc gccaaacgca ccggtgatgg tacccatctc 1560

tgggattgcg ccgataagaa taataacgat gttgttcaaa ccaaagcgca aatcgatacc 1620

ctctgcacca aacatgagta cccctacgac gtgcccgact acgcctaaca tatgccatgg 1680

tgtcaaaacc ggggttagtg acattgactt gttgacaaaa atctgtatag ctagaaaact 1740

ctaagcaacg cttttctttg ttttattttt tatgtttaaa ctccttcaga attgtaggat 1800

atcttgtttt gaaaaatcca ggactgagtt tcgttgcccc atttgcttgt tctcgtttga 1860

aatgtcgaac aatagaaatg cttgcagaat gaggttctcc tttacaaaaa aactcgatag 1920

ggttcaatat gaagctgtct caatgcatag atttccacga ttttaccttt gcataatcta 1980

tggtgcgcgt cagatgccac cctcgtcgct gtacaaccaa tacattgtag cttcattttg 2040

acattaggta ccttcttccc cgacctcctt cagaatctca gagtaagcga tcgtcacccc 2100

ttctacctga aactctacca ctgcatacgt agtaaaggcc tctaattacc acggtagtac 2160

tattcttgca ctgaggaatt ctctagacga atgtaggcta ttcttaatgg accggccctc 2220

agctcgatta tttttgcttg acttgacttg acttgattca tgaagttgat aggaaagaaa 2280

cataacccat cccatcccac aacctgcgtg tactctgatc ggcaggtgca cgctgagttg 2340

aaggtggttc aagaatcgaa aacatcagcc tagagcacga cgaggtttca gagagccaac 2400

tttttctatc tattaatctc atcctttgct tcttcgcgga caacgacggt ggatcagcgc 2460

cgccgctgag aagacagcag aggtaactct agcaagagaa gcagcagtag cttcgtctgg 2520

tcaagagact ctgcttaagc acagtagcct gcaaataaag acacttgggc aaaagaaaca 2580

ttgacattga ttgaatttca cgcagaggca aatggaagct t 2621

<210> 9
<211> 9
<212> PRT
<213> Artificial sequence

<220>
<223> HA tag sequence

<400> 9

Tyr Pro Tyr Asp Val Pro Asp Tyr Ala
1               5

<210> 10
<211> 35
<212> DNA
<213> Artificial sequence

<220>
<223> Primer

<400> 10
ttgagctcag agcgcgaaag agagtgccgg aattc 35

<210> 11
<211> 35
<212> DNA
<213> Artificial sequence

<220>
<223> Primer

<400> 11
gcggatccga aattgacctt actgcctctc ctgtg 35

<210> 12
<211> 35
<212> DNA

<213>  Artificial sequence

<220>
<223>  Primer

<400>  12
gggcatgctg tcaaaaccgg ggttagtgac attga                                35


<210>  13
<211>  35
<212>  DNA
<213>  Artificial sequence

<220>
<223>  Primer

<400>  13
ggaagcttcc atttgcctct gcgtgaaatt caatc                                35


<210>  14
<211>  34
<212>  DNA
<213>  Artificial sequence

<220>
<223>  Primer

<400>  14
gcggatccat ggccaagttg accagtgccg ttcc                                 34


<210>  15
<211>  34
<212>  DNA
<213>  Artificial sequence

<220>
<223>  Primer

<400>  15
gcgtcgactc agtcctgctc ctcggccacg aagt                                 34


<210>  16
<211>  2163
<212>  DNA
<213>  Artificial sequence

<220>
<223>  Optimized sequence for the expression of the zeocin resistance in
       Aurantiochytrium

<400>  16
gagctcagag cgcgaaagag agtgccggat tcaaagacgc cacagcggga aagaaagaaa      60

gacctaggag gtactagctg gttgtagcta gctagctagc tagctagctt atgctgctaa     120

gacgcccttc ctcctcgagg tccttttgac ttgccagcgc agtctccttt gtcttcttcg     180

ctcatttaat caagtcaagt cttcaggttt aaaatgaaaa atcctgcttc caggttcagt     240

tctagcaagt aggtaggtgg cagtagcgtt acgaggagga gtcccgagag ggagtcggag 300
agtagaaaac tggaagtcgg cgaaacaaaa ggcgcagaga tttgccggaa tggagagtta 360
tcgtgagact ctctgagtag acccaagtgt cctgtgaggc actcgtgata gggagggggc 420
acgggctgaa gggggctaca gtaaggagag agtggcgtca gtggagtttc gccgagaact 480
cttcgagaaa gaggaagaga ggaaccgaga gcgccgttga agaggggaaa aagcagacgg 540
tttaattata attaattaag taattaatta cttacttatt gattgattga tttgagaaga 600
gaagcaaaga gagagttgaa gaaatagtaa cgaagaatag gagaagaaag gggcaagaaa 660
agaaaaaaga aagaggagaa tattggtcga tgagcgagaa cgtgcaaatc caaaacagca 720
aaactcaaac tcaaactaca agaagcgtgg cgttgcagag gcaacagctc gaaagcaaca 780
cagaacagac aaacacagga gaggcagtaa ggtcaatttc ggatccatgg ccaagttgac 840
cagtgccgtt ccggtgctca ccgcgcgcga cgtcgccgga gcggtcgagt tctggaccga 900
ccggctcggg ttctcccggg acttcgtgga ggacgacttc gccggtgtgg tccgggacga 960
cgtgaccctg ttcatcagcg cggtccagga ccaggtggtg ccggacaaca ccctggcctg 1020
ggtgtgggtg cgcggcctgg acgagctgta cgccgagtgg tcggaggtcg tgtccacgaa 1080
cttccgggac gcctccgggc cggccatgac cgagatcggc gagcagccgt gggggcagga 1140
gttcgccctg cgcgacccgg ccggcaactg cgtgcacttc gtggccgagg agcaggactg 1200
agtcgacctg caggcatgct gtcaaaaccg gggttagtga cattgacttg ttgacaaaaa 1260
tctgtatagc tagaaaactc taagcaacgc ttttctttgt tttatttttt atgttttaac 1320
tccttcagaa ttgtaggata tcttgttttg aaaaatccag gactgagttt cgttgcccca 1380
tttgcttgtt ctcgtttgaa atgtcgaaca gtagaaatgc ttgcagaatg aggttctcct 1440
ttacaaaaaa ctcgataggg ttcaatatga agctgtctcg atgcatagat ttccacgatt 1500
ttacctttgc ataatctatg gtgcgcgtca gatgccaccc tcgtcgctgt acaaccaata 1560
cattgtagct tcattttgac attaggtacc ttcttccccg acctccttca gaatctcaga 1620
gtaagcgatc gatcgtcacc ccttctacct gaaactctac cactgcatac gtagtaaagg 1680
cctctaatta ccacggtagt actattcttg cactgaggaa ttctctagac gaatgtaggc 1740
tattcttaat ggaccggccc tcagctcgat tatttttgct tgacttgact tgacttgatt 1800
aatgaagttg ataggaaaga aacataaccc attccatccc acaacctgcg tgtactctga 1860
tcggcaggtg cacgctgagt tgagggtgat ttaaggatcg aaaacatcag cctagagcac 1920
gacgaggttc cagagagcca actttttcta tctattaatc tcatcctttg cttcttcgcg 1980
gacaacgacg gtggatcagc gccgccgctg agaagacagc agaggtaact ctagcaagag 2040
aagcagcagt agcttcgtct ggtcaagaga ctctgcttaa gcacagtagc ctgcaaataa 2100
agacacttgg gcaaaagaaa cattgacatt gattgaattt cacgcagagg caaatggaag 2160

ctt                                                                    2163

<210>  17
<211>  2370
<212>  DNA
<213>  Artificial sequence

<220>
<223>  Optimized sequence for the expression of a delta11 desaturase in
       Aurantiochytrium

<400>  17
ctgcaggtag gtaggtggca gtagcgttac gaggaggagt cccgagaggg agtcggagag      60

tagaaaactg gaagtcggcg aaacaaaagg cgcagagatt tgccggaatg gagagttatc     120

gtgagactct ctgagtagac ccaagtgtcc tgtgaggcac tcgtgatagg gagggggcac    180

gggctgaagg gggctacagt aaggagagag tggcgtcagt ggggttttgc cgagaactct      240

tcgagaaaga ggaagagagg aaccgagagc gccgttgaag aggggaaaaa gcagacggtt     300

taattataat taattaagta attaattact tacttattga ttgattgatt tgagaagaga     360

agcaaagaga gagttgaaga aatagtaacg aagaatagga gaagaaaggg gcaagaaaag     420

aaaaagaaag aggagaatat tagtcgatga gcgagaacgt gcaaatccaa aacagcaaaa     480

ctcaaactca aactcaaact acaagaagcg tggcgttgca gaggcaacag ctcgaaagca     540

acacagaaca aacaaacaca ggagaggcag taaggtcaat ttcgcggccg cgctagcatg     600

gcgcccaaca cgcgggagaa cgagacgatc tacgacgaag tggaacacaa gctggagaag     660

ctcgtgcccc cccaggcggg cccctggaac tacaagatcg tgtacctgaa cttgctgacc     720

ttctcctact ggctgatcgc cggcgcctac gggttgtact tgtgcttcac gtccgccaag     780

tgggccacga tcatcttcga attcatcttg ttcttcttcg ccgagatggg catcacggcc     840

ggcgcccacc ggctgtggac gcacaagtcc tacaaggcca agttgccctt ggaaatcttc     900

ctcatggtgc tgaactccgt ggcgttccag aacacggcca ccgactgggt gcgggaccac     960

cggctgcacc acaagtacag cgacacggac gcggaccccc acaacgccgc gcgggggctg    1020

ttcttctccc acgtcgggtg gctgctcgtc cggaagcacg acgaagtcaa gaagcgcggg    1080

aagttcaccg acatgtccga catctacaac aaccccgtgt tgaagttcca gaagaagtac    1140

gccatcccct tcatcggcgc cgtgtgcttc atcttgccca cggtgatccc catgtacttc    1200

tggggcgagt ccctcaacaa cgcctggcac atctgcatcc tgcggtacgc gatgaacctc    1260

aacgtcacgt tctccgtgaa ctccctggcg cacatctggg gcaacaagcc ctacgacaag    1320

gacatcaagc ccgcccagaa cttcggcgtg acgttggcga ccttcggcga agggttccac    1380

aactaccacc acgtgttccc ctgggactac cggacgtccg aactcggcga caacaagttc    1440

aacttcacga cgaagttcat caacttcttc gaacggatcg gcttggcgta cgacctgaag    1500

accgtgtccg acgacgtgat cgcgcagcgg gccaagcgga ccggcgacgg cacgcacctg 1560

tgggactgcg ccgacaagaa caacaacgac gtggtgcaga cgaaggcgca gatcgacacc 1620

ttgtgcacga agcacgaatg aggttctcct ttacaaaaaa actcgatagg gttcaatatg 1680

aagctgtctc aatgcataga tttccacgat tttacctttg cataatctat ggtgcgcgtc 1740

agatgccacc ctcgtcgctg tacaaccaat acattgtagc ttcattttga cattaggtac 1800

cttcttcccc gacctccttc agaatctcag agtaagcgat cgtcacccct tctacctgaa 1860

actctaccac tgcatacgta gtaaaggcct ctaattacca cggtagtact attcttgcac 1920

tgaggaattc tctagacgaa tgtaggctat tcttaatgga ccggccctca gctcgattat 1980

ttttgcttga cttgacttga cttgattcat gaagttgata ggaaagaaac ataacccatc 2040

ccatcccaca acctgcgtgt actctgatcg gcaggtgcac gctgagttga aggtggttca 2100

agaatcgaaa acatcagcct agagcacgac gaggtttcag agagccaact ttttctatct 2160

attaatctca tcctttgctt cttcgcggac aacgacggtg gatcagcgcc gccgctgaga 2220

agacagcaga ggtaactcta gcaagagaag cagcagtagc ttcgtctggt caagagactc 2280

tgcttaagca cagtagcctg caaataaaga cacttgggca aaagaaacat tgacattgat 2340

tgaatttcac gcagaggcaa atggaagctt 2370

## Description

The invention pertains to a method for the production of triacylglycerides (TAGs or Triacylglycerols) and fatty acids by the recombinant expression of a Δ11 fatty acid desaturase in protists.

Polyunsaturated fatty acids (PUFAs), such as the ω3-docosahexaenoic acid (DHA, 22:6) and the ω3-eicosapentaenoic acid (EPA, 20:5), have multiple benefits for human health (Simopoulos AP, Experimental Biology and Medicine, 233(6):674-88, 2008). In particular, DHA plays a crucial role in various biochemical processes and is necessary to the normal functional development of cells (for example, DHA is necessary for brain development in newborns and children). However, PUFAs are poorly synthesized in animals and are thus considered as essential fatty acids, which must be obtained by diet. Today, the most widely and naturally available diet source is fish oil but the overexploitation of fish stocks and the contamination by toxic substances (such as heavy metals) impose to find viable alternative sources. Since fishes obtain their ω3-fatty acids from zooplankton that consumes phytoplankton, microalgae and marine protists, like Thraustochytrids, appear to be a promising source of ω3-PUFAs (Ward OP & Singh A, Process Biochemistry, 40(12):3627-52, 2005; Adrame-Vega TC et al., Current Opinion in Biotechnology, 26:14-8, 2014).

The yields of TAGs and of PUFAs contained in these TAGs may considerably vary from one protist to another and also depend on the growth conditions. It is well known that nutrient-deprived growth media (especially nitrogen or phosphorus deprived media) trigger lipid accumulation in microalgae species such as *Phaeodactylum tricornutum* or *Nannochloropsis gaditana* (Jouhet J et al., PLoS One, 12(8):e0182423, 2017). However, nutrient deficiencies in these organisms are associated with a growth arrest and an accumulation of TAGs, often at the expense of membrane glycerolipids so that the total amount of lipids per gram of biomass does not increase.

To overcome this problem, many attempts are made on various algal models to engineer the biosynthesis of fatty acids in order to get high lipid levels in fast growing cell cultures with a high biomass. So far, best results were obtained by overexpressing a recombinant enzyme diacylglycerol acyltransferase, which is directly involved in TAG synthesis, leading to a doubling of the total fatty acid content with only a moderate decrease of the growth rate (Dinamarca J et al., Journal of Phycology, 53(2):405-14, 2017). However, the above and most widely-used algal models are very poor in DHA, which has a recognized nutritional importance and a strong potential in terms of therapeutic applications.

Today, only few attempts have been made to engineer Thraustochytrids (Aasen IM et a/., Applied Microbiology and Biotechnology, 100(10):4309-21, 2016; Yan JF et al., Applied Microbiology and Biotechnology, 97(5):1933-9, 2013) with only limited effects on the TAGs and ω3-fatty acid production. For example, a Δ5 desaturase was overexpressed to increase EPA in Thraustochytrids, but addition in the external medium of the substrate of the enzyme (ETA, 20:4) is required to obtain some EPA production (Kobayashi T et al., Applied and Environmental Microbiology, 77(11):3870-6, 2011).

Therefore, there is a need for new tools to increase the production of TAGs and fatty acids with these microorganisms.

In this context, the Inventors have found that the expression of a recombinant Δ11 fatty acid desaturase from insect results in a higher rate of growth in protists, thus improving the biomass production, together with an increase of total fatty acids and TAGs, without affecting the fatty acid composition. Moreover, no exogenous lipid precursor is needed in the culture medium to trigger fatty acids and TAGs accumulation.

In an aspect, the invention thus relates to the use of a recombinant Δ11 fatty acid desaturase comprising or consisting of a sequence having at least 50% identity with the sequence SEQ ID NO: 1 for increasing the content of triacylglycerides and/or the content of fatty acids in a protist.

More specifically, the invention provides a method for producing triacylglycerides and/or fatty acids, wherein said method comprises a step of expression of a recombinant fatty acid Δ11 desaturase comprising or consisting of a sequence having at least 50% identity with the sequence SEQ ID NO: 1 in a protist.

The main benefits of the method of the invention are found in the fact that it induces not only an increase of the biomass of the cultivated protist but also an increase of the content of total TAGs and fatty acids per cell.

As used herein, the term "triacylglyceride" (TAG or Triacylglycerol) refers to a lipid consisting of three fatty acids esterified to glycerol. In a triacylglyceride, the glycerol may be linked to saturated and/or unsaturated fatty acids. The triacylglycerides produced in the invention preferably contain one, two or three unsaturated fatty acids. More preferred are triacylglycerides containing one, two or three polyunsaturated fatty acids.

Preferably, the fatty acids which are produced in the invention are polyunsaturated fatty acids.

As used herein, the term "polyunsaturated fatty acid" (PUFA) refers to a fatty acid (*i.e.* a carboxylic acid with an aliphatic chain) that contains more than one double bond in its backbone. PUFAs are derived from fatty acids with 4 to 22 carbon atoms. Preferably, the PUFAs produced in the invention are long chain polyunsaturated fatty acids (LCPUFA) which are derived from fatty acids with 16 to 22 carbon atoms. More preferably, the PUFAs produced in the invention are very long chain polyunsaturated fatty acids (VLCPUFA) which are derived from fatty acids with 20 to 22 carbon atoms.

The PUFAs produced in the invention may be bound in membrane lipids and/or in TAGs, but they may also occur as free fatty acids or else bound in the form of other fatty acid esters. In this context, they may be present as pure products or in the form of mixtures of various fatty acids or mixtures of different glycerides.

In the invention, the PUFAs as free fatty acids or bound in the TAGs have preferably a chain length of at least 16 carbon atoms, more preferred are LCPUFA and VLCPUFA, even more preferred are eicosapentaenoic acid (EPA, 20:5), docosapentaenoic acid (DPA, 22:5), or docosahexaenoic acid (DHA, 22:6).

As used herein, "Eicosapentaenoic acid" (EPA) designates a PUFA which contains 20 carbons and 5 double bonds (20:5).

As used herein, "Docosapentaenoic acid" (DPA) designates a PUFA which contains 22 carbons and 5 double bonds (22:5).

As used herein, "Docosahexaenoic acid" (DHA) designates a PUFA which contains 22 carbons and 6 double bonds (22:6).

As used herein, the term "Δ11 fatty acid desaturase" (or delta11 fatty acid desaturase) refers to an enzyme which is capable of introducing a double bond at the 11^{th} position from the carboxyl end into fatty acids or their derivatives, such as fatty acyl-CoA esters. In particular, the Δ11 fatty acid desaturase used in the invention is a Δ11 acyl-CoA desaturase, which means it is capable of using acyl-CoA fatty acids as substrate. More preferred is a Δ11 acyl-CoA desaturase that is capable of desaturating acyl-CoA molecules with a chain length of 16 carbons or more.

In the method of the invention, the amino acid sequence of the recombinant Δ11 fatty acid desaturase expressed in a protist is an exogenous enzyme which may originate from insects, in particular from moths.

Indeed, the desaturases expressed in pheromone glands of different moth species play a key role in the biosynthesis of sex pheromones, exhibiting a wide variety of substrate and region- and stereo-specificities. In particular, pheromone gland desaturases catalyze the formation of uncommon unsaturated fatty acyl-CoA esters with variable chain lengths and either the ordinary Z or the unusual E double bond geometry.

Preferably, the amino acid sequence of the Δ11 fatty acid desaturase used in the invention originates from the Lepidoptera family, in particular selected from the group consisting of *Acrolepiidae, Agaristidae, Arctiidae, Bombycidae, Carposinidae, Cochylidae, Cossidae, Eriocraniidae, Gelechiidae, Geometridae, Gracillariidae, Hepialidae, Ithomiidae, Lasiocampidae, Lycaenidae, Lymantriidae, Lyonetiidae, Nepticulidae, Noctuidae, Notodontidae, Nymphalidae, Oecophoridae, Papilionidae, Pieridae, Psychidae, Pterophoridae, Pyralidae, Saturniidae, Sesiidae, Sphingidae, Tortricidae, Yponomeutidae* and *Zygaenidae.* More preferably, the amino acid sequence of the Δ11 fatty acid desaturase used in the invention originates from the genera *Thaumetopoea, Helicoverpa* or *Spodoptera,* and in particular selected from the species *Thaumetopoea pityocampa, Helicoverpa zea* or *Spodoptera littoralis.*

In an embodiment, the recombinant Δ11 fatty acid desaturase comprises or consists of a sequence having at least 50%, 51%, 52%, 53%, 54%, 55%, 56%, 57%, 58%, 59%, 60%, 61%, 62%, 63%, 64%, 65%, 66%, 67%, 68%, 69%, 70% 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with the sequence SEQ ID NO: 1.

As used herein, the "percentage identity" (or "% identity") between two sequences of nucleic acids or amino acids means the percentage of identical nucleotides or amino acid residues between the two sequences to be compared, obtained after optimal alignment, this percentage being purely statistical and the differences between the two sequences being distributed randomly along their length. The comparison of two nucleic acid or amino acid sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman, by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr., Madison, Wl, or by the comparison software BLAST NR or BLAST P). The percentage identity between two nucleic acid or amino acid sequences is determined by comparing the two optimally-aligned sequences in which the nucleic acid or amino acid sequence to compare can have insertions or deletions compared to the reference sequence for optimal alignment between the two sequences. Percentage identity is calculated by determining the number of positions at which the amino acid, nucleotide or residue is identical between the two sequences, preferably between the two complete sequences, dividing the number of identical positions by the total number of positions in the alignment window and multiplying the result by 100 to obtain the percentage identity between the two sequences.

In an embodiment, the recombinant fatty acid Δ11 desaturase comprises or consists of a sequence selected from the group comprising SEQ ID NO: 1, SEQ ID NO: 2 and SEQ ID NO: 3, preferably SEQ ID NO: 1.

**Table 1. Amino acid sequences of three reference Δ11 Acyl-CoA desaturases from Thaumetopoea pityocampa, Helicoverpa zea or Spodoptera littoralis respectively.**

| | |
|---|---|
| **SEQ ID NO: 1** Δ11 Acyl-CoA desaturase from *Thaumetopoea pityocampa* (UniProt A8QVZ1) | MAPNTRENETIYDEVEHKLEKLVPPQAGPWNYKIVYLNLLTFSYWLI AGAYGLYLCFTSAKWATIIFEFILFFFAEMGITAGAHRLWTHKSYKA KLPLEIFLMVLNSVAFQNTATDWVRDHRLHHKYSDTDADPHNAARGL FFSHVGWLLVRKHDEVKKRGKFTDMSDIYNNPVLKFQKKYAIPFIGA VCFILPTVIPMYFWGESLNNAWHICILRYAMNLNVTFSVNSLAHIWG NKPYDKDIKPAQNFGVTLATFGEGFHNYHHVFPWDYRTSELGDNKFN FTTKFINFFERIGLAYDLKTVSDDVIAQRAKRTGDGTHLWDCADKNN NDVVQTKAQIDTLCTKHE |
| **SEQ ID NO: 2** Δ11 Acyl-CoA desaturase from *Helicoverpa zea* (UniProt Q9NB26) | MAQSYQSTTVLSEEKELTLQHLVPQASPRKYQIVYPNLITFGYWHIA GLYGLYLCFTSAKWATILFSYILFVLAEIGITAGAHRLWAHKTYKAK LPLEILLMVFNSIAFQNSAIDWVRDHRLHHKYSDTDADPHNASRGFF YSHVGWLLVRKHPEVKKRGKELNMSDIYNNPVLRFQKKYAIPFIGAV CFALPTMIPVYFWGETWSNAWHITMLRYIMNLNVTFLVNSAAHIWGN KPYDAKILPAQNVAVSVATGGEGFHNYHHVFPWDYRAAELGNNSLNL TTKFIDLFAAIGWAYDLKTVSEDMIKQRIKRTGDGTDLWGHEQNCDE VWDVKDKSS |
| **SEQ ID NO: 3** Δ11 Acyl-CoA desaturase from *Spodoptera littoralis* (UniProt Q6US81) | MAQCVQTTTILEQKEEKTVTLLVPQAGKRKFEIVYFNIITFAYWHIA GLYGLYLCFTSTKWATVLFSFFLFVVAEVGVTAGSHRLWSHKTYKAK LPLQILLMVMNSLAFQNTVIDWVRDHRLHHKYSDTDADPHNASRGFF YSHVGWLLVRKHPDVKKRGKEIDISDIYNNPVLRFQKKYAIPFIGAV CFVLPTLIPVYGWGETWTNAWHVAMLRYIMNLNVTFLVNSAAHIYGK RPYDKKILPSQNIAVSIATFGEGFHNYHHVFPWDYRAAELGNNSLNF PTKFIDFFAWIGWAYDLKTVSKEMIKQRSKRTGDGTNLWGLEDVDTP EDLKNTKGE |

In an embodiment, the sequence of the recombinant Δ11 fatty acid desaturase used in the invention contains three highly conserved His-rich boxes consisting of SEQ ID NO: 4 (HRLW[T/A/S]H), SEQ ID NO: 5 ([D/E]HR[L/M/F/S]HH[K/R]) and SEQ ID NO: 6 ([F/S]HNYHH[V/T]) respectively.

In a particular embodiment, the recombinant Δ11 fatty acid desaturase can be in the form of a fragment (or a truncated sequence) of a sequence having at least 50% identity with SEQ ID NO: 1. Such a fragment of the Δ11 fatty acid desaturase preferably exhibits the same, or substantially the same, activity compared to the full length Δ11 fatty acid desaturase.

The desaturase activity can be verified by cultivating the microorganism expressing the recombinant enzyme, digesting it in a suitable buffer or solvent, bringing the digest into contact with fatty acids or acyl-CoA fatty acids and, if appropriate, with a cofactor such as NADH or NADPH or oxygen, and detecting the resulting desaturated fatty acids or acyl-CoA fatty acids. The fatty acids or acyl-CoA fatty acids can preferably originate from the transformed organism if it is itself capable of synthesizing fatty acids or acyl-CoA fatty acids. If not, however, it is also possible to add fatty acids or acyl-CoA fatty acids. The fatty acid or acyl-CoA fatty acid which has been modified by the desaturase or conjugase can be detected via customary methods with which the skilled work is familiar, if appropriate after extraction from the incubation mixture, for example with a solvent such as ethyl acetate. Separation methods such as high-performance liquid chromatography (HPLC), gas chromatography (GC), thin-layer chromatography (TLC) and detection methods such as mass spectroscopy (MS or MALDI), UV spectroscopy or autoradiography may be employed for this purpose.

In the invention, the expression of the recombinant Δ11 fatty acid desaturase takes place in protists.

As used herein, the term "protists" refers to the one-celled eukaryotic microorganisms classified in the taxonomic kingdom Protista. Protists are not animals, plants, fungi, yeast or bacteria. In the invention, suitable protists are autotroph or heterotroph, preferably heterotroph.

In an embodiment, the expression of the recombinant Δ11 fatty acid desaturase of the invention takes place in a protist which is a microalgae.

As used herein, the expression "microalgae" refers to microscopic algae, with sizes from a few micrometers to a few hundred micrometers.

In particular, the expression "microalgae" covers the microalgae with high industrial potential (for example used as food supplements or used for biofuel production) : such as *Nannochloropsis gatidana, Phaeodactylum tricornutum* and *Thalassiosira pseudonana.*

In an embodiment, the expression of the recombinant Δ11 fatty acid desaturase of the invention takes place in a protist which is selected from the phylogenetic group SAR.

In an embodiment, the expression of the recombinant Δ11 fatty acid desaturase of the invention takes place in a protist which is selected from the supergroup Chromalveolata (Adl SM et al., Journal of Eukaryotic Microbiology, 52(5):399-451, 2005).

In an embodiment, the expression of the recombinant Δ11 fatty acid desaturase of the invention takes place in a protist which is a traustochytrid (*Thraustochytriidae* family), preferably from a genus selected from the group consisting of *Aurantiochytrium, Japonochytrium, Sicyoidochytrium, Ulkenia, Parietichytrium, Botryochytrium, Schizochytrium, Monorhizochytrium* and *Thraustochytrium.*

In particular, *Aurantiochytrium* is a thraustochytrid genus defined by Yokohama and Honda in 2007 (Yokoyama R. & Honda D, Mycoscience, 48:199-211, 2007, which is incorporated herein by reference for the purpose of defining the genus *Aurantiochytrium,* in particular last paragraph of page 207).

The genus *Aurantiochytrium* is characterized by the absence of well-developed ectoplasmic nets and a lower number of zoospores produced by each zoosporangium compared to the genus *Schizochytrium.* Molecular analyses of the 18S rDNA region and chemotaxonomical observations has revealed a clear separation of the two taxa. Moreover, *Schizochytrium* only synthesizes β-carotene as main pigment and between 15 and 30% of arachidonic acid (AA 20:4 ω6), whereas *Aurantiochytrium* can produce besides β-carotene, astaxantin, cantaxanthin and its intermediates and the main fatty acid is DHA with very low levels of AA.

Preferably, the expression of the recombinant Δ11 fatty acid desaturase of the invention takes place in a protist selected from the species *Aurantiochytrium limacinum* and *Aurantiochytrium mangrovei.*

The invention is preferably carried out in *Auranthiochytrium limacinum* (formerly *Schizochytrium limacinum*), a heterotrophic marine protist naturally rich in DHA (> 30-40% of total fatty acids), which emerged as a micro algal model.

In a particular embodiment, the invention relates to a method for producing triacylglycerides and/or fatty acids, wherein said method comprises a step of expression of a recombinant fatty acid Δ11 desaturase comprising or consisting of a sequence having at least 50%, 51%, 52%, 53%, 54%, 55%, 56%, 57%, 58%, 59%, 60%, 61%, 62%, 63%, 64%, 65%, 66%, 67%, 68%, 69%, 70% 71%, 72%, 73%, 74%, 75%, 76%, 77%, 78%, 79%, 80%, 81%, 82%, 83%, 84%, 85%, 86%, 87%, 88%, 89%, 90% , 91%, 92%, 93%, 94%, 95%, 96%, 97%, 98%, 99% or 100% identity with the sequence SEQ ID NO: 1, in a Thraustochytrid, preferably from a genus selected from the group consisting of *Aurantiochytrium, Japonochytrium, Sicyoidochytrium, Ulkenia, Parietichytrium, Botryochytrium, Schizochytrium, Monorhizochytrium* and *Thraustochytrium,* more preferably from the species *Aurantiochytrium limacinum.*

In an embodiment, the invention relates to the method as defined above, wherein the fatty acids are polyunsaturated fatty acids, which have preferably a chain length of 16 carbons or more.

In an embodiment, the invention relates to the method as defined above, wherein the polyunsaturated fatty acids have a chain length of 16, 18, 20 or 22 carbons.

In an embodiment, the invention relates to the method as defined above, wherein the polyunsaturated fatty acids are ω3-polyunsaturated fatty acids.

As used herein, "ω3-potyunsaturated fatty acids" (ω3-PUFAs or omega3-polyunsaturated fatty acids) are PUFAs with a double bond at the third carbon atom from the methyl end of the carbon chain.

In an embodiment, the invention relates to the method as defined above, wherein the ω3-PUFAs have a chain length of 16, 18, 20 or 22 carbons.

Preferred ω3-PUFAs are EPA, DPA and DHA.

In an embodiment, the invention relates to the method as defined above, wherein the TAGs contain at least one ω3-PUFA, said at least one ω3-PUFA having preferably 16 carbons or more.

In an embodiment, the invention relates to the method as defined above, wherein the TAGs contain at least one w3-PUFA, said at least one ω3-PUFA being preferably selected from EPA, DPA and DHA.

In the invention, the content of TAGs and fatty acids in the protist cell expressing the recombinant Δ11 fatty acid desaturase is increased compared to the content of TAGs and fatty acids in the wild type protist cell grown in the same conditions.

In particular, the quantity of TAGs per cell (or per liter of culture or per liter of culture per day) of the protist expressing the recombinant Δ11 fatty acid desaturase is increased by at least a factor 1.1 compared to the quantity of TAGs per cell (or per liter of culture or per liter of culture per day) of the wildtype protist. Preferably the production of TAGs in the protist expressing the recombinant Δ11 fatty acid desaturase is increased by a factor 1.5, 2.0, 2.5, 3.0 or higher.

In particular, the quantity of fatty acids per cell (or per liter of culture or per liter of culture per day) of the protist expressing the recombinant Δ11 fatty acid desaturase is increased by at least a factor 1.1 compared to the quantity of fatty acids per cell (or per liter of culture or per liter of culture per day) of the wildtype protist. Preferably the production of fatty acids in the protist expressing the recombinant Δ11 fatty acid desaturase is increased by a factor 1.5, 2.0, 2.5, 3.0 or higher.

In an embodiment, the content of PUFAs in the protist cell expressing the recombinant Δ11 fatty acid desaturase is increased compared to the content of PUFAs in the wild type protist cell grown in the same conditions.

In particular, the quantity of PUFAs per cell (or per liter of culture or per liter of culture per day) of the protist expressing the recombinant Δ11 fatty acid desaturase is increased by at least a factor 1.1 compared to the quantity of PUFAs per cell (or per liter of culture or per liter of culture per day) of the wildtype protist. Preferably the production of PUFAs in the protist expressing the recombinant Δ11 fatty acid desaturase is increased by a factor 1.5, 2.0, 2.5, 3.0 or higher.

In particular, the quantity of DHA per cell (or per liter of culture or per liter of culture per day) of the protist expressing the recombinant Δ11 fatty acid desaturase is increased by at least a factor 1.1 compared to the quantity of DHA per cell (or per liter of culture or per liter of culture per day) of the wildtype protist. Preferably the production of DHA in the protist expressing the recombinant Δ11 fatty acid desaturase is increased by a factor 1.5, 2.0, 2.5, 3.0 or higher.

In particular, the quantity of DPA per cell (or per liter of culture or per liter of culture per day) of the protist expressing the recombinant Δ11 fatty acid desaturase is increased by at least a factor 1.1 compared to the quantity of DPA per cell (or per liter of culture or per liter of culture per day) of the wildtype protist. Preferably the production of DPA in the protist expressing the recombinant Δ11 fatty acid desaturase is increased by a factor 1.5, 2.0, 2.5, 3.0 or higher.

In particular, the quantity of EPA per cell (or per liter of culture or per liter of culture per day) of the protist expressing the recombinant Δ11 fatty acid desaturase is increased by at least a factor 1.1 compared to the quantity of EPA per cell (or per liter of culture or per liter of culture per day) of the wildtype protist. Preferably the production of EPA in the protist expressing the recombinant Δ11 fatty acid desaturase is increased by a factor 1.5, 2.0, 2.5, 3.0 or higher.

Another benefit of the present invention is that the expression of the recombinant Δ11 fatty acid desaturase in a protist induces not only an increase of the production of TAGs and fatty acids per cell but also an increase of the growth of the protist, and thus an increase of the biomass produced after culture.

In particular, a protist expressing the recombinant Δ11 fatty acid desaturase has a higher rate of growth compared to the wild type protist. For example, after 5 days of culture, the biomass of the protist expressing the recombinant Δ11 fatty acid desaturase is increased by at least a factor 1.1, preferably at least a factor 1.5, compared to the biomass of the wild type protist grown in the same conditions.

In an embodiment, the invention relates to a method as defined above, which further comprises a step of culture of the protist expressing the recombinant fatty acid Δ11 desaturase.

The culture of the protists is generally carried out in heterotrophic mode, preferably in chemically defined media. Some chemically defined culture media that can be used in the invention contain a carbon source, a nitrogen source and salts necessary to microorganism growth. The person skilled in the art knows well the elements necessary to microorganism growth.

For example, Traustochytrids, such as *Auranthiochytrium limacinum,* can be cultivated in a R medium as defined in Table 5 in the examples.

*Auranthiochytrium limacinum* is generally cultivated at a temperature between 20°C and 30°C, preferably at 25°C.

*Auranthiochytrium limacinum* can also be cultivated at low temperatures, such as 15°C, since some studies have taught that low temperatures can increase its production of DHA.

In an embodiment, the invention relates to the method as defined above, wherein said method further comprises a step of lipid extraction from the culture of the protist expressing the recombinant fatty acid Δ11 desaturase.

After the protists have been cultured, the lipids are obtained in the customary manner. To this end, the protists can first be digested or else used directly. The lipids are advantageously extracted with suitable solvents such as apolar solvents, such as hexane or ethanol, isopropanol or mixtures such as hexane/isopropanol, phenol/chloroform/isoamyl alcohol, at temperatures between 0°C to 80°C, preferably between 20°C and 50°C. Some appropriate methods are presented in the examples.

In another aspect, the invention relates to oils, fatty acid mixtures and/or TAG mixtures, in particular with an increased content of PUFAs, which have been produced by the above-described method, and to their use for the production of foodstuffs, feedstuffs, cosmetics or pharmaceuticals. To this end, they are added in customary amounts to the foodstuffs, feedstuffs, cosmetics or pharmaceuticals.

In another aspect, the invention relates to a nucleic acid encoding a fatty acid Δ11 desaturase comprising or consisting of a sequence having at least 50% identity with the sequence SEQ ID NO: 1, said nucleic acid being codon-optimized for the expression of said fatty acid Δ11 desaturase in a protist.

In an embodiment, the invention relates to a nucleic acid encoding a fatty acid Δ11 desaturase comprising or consisting of a sequence having at having at least 50%, 51%, 52%, 53%, 54%, 55%, 56%, 57%, 58%, 59%, 60%, 61%, 62%, 63%, 64%, 65%, 66%, 67%, 68%, 69%, 70% 71%, 72%, 73%, 74%, 75%, 76%, 77%, 78%, 79%, 80%, 81%, 82%, 83%, 84%, 85%, 86%, 87%, 88%, 89%, 90% , 91%, 92%, 93%, 94%, 95%, 96%, 97%, 98%, 99% or 100% identity with the sequence SEQ ID NO: 1, said nucleic acid being codon-optimized for the expression of said fatty acid Δ11 desaturase in a protist.

In an embodiment, the invention relates to a nucleic acid encoding a fatty acid Δ11 desaturase comprising or consisting of a sequence having at least 50% identity with the sequence SEQ ID NO: 1, said nucleic acid being codon-optimized for the expression of said fatty acid Δ11 desaturase in a microalgae.

In an embodiment, the invention relates to a nucleic acid encoding a fatty acid Δ11 desaturase comprising or consisting of a sequence having at least 50% identity with the sequence SEQ ID NO: 1, said nucleic acid being codon-optimized for the expression of said fatty acid Δ11 desaturase in a protist which is selected from the phylogenetic group SAR, which comprises Stramenopiles, Alveolates and Rhizaria (Burki F et a/., PLoS One. 2(8):e790, 2007).

In an embodiment, the invention relates to a nucleic acid encoding a fatty acid Δ11 desaturase comprising or consisting of a sequence having at least 50% identity with the sequence SEQ ID NO: 1, said nucleic acid being codon-optimized for the expression of said fatty acid Δ11 desaturase in a protist which is selected from the supergroup Chromalveolata.

In an embodiment, the invention relates to a nucleic acid encoding a fatty acid Δ11 desaturase comprising or consisting of a sequence having at least 50% identity with the sequence SEQ ID NO: 1, said nucleic acid being codon-optimized for the expression of said fatty acid Δ11 desaturase in a Traustochytrid, preferably from a genus selected from the group consisting of *Aurantiochytrium, Japonochytrium, Sicyoidochytrium, Ulkenia, Parietichytrium, Botryochytrium, Schizochytrium, Monorhizochytrium* and *Thraustochytrium.* more preferably from the species *Aurantiochytrium limacinum* and *Aurantiochytrium mangrovei.*

In an embodiment, the invention relates to a nucleic acid as defined above which comprises or consists of the sequence SEQ ID NO: 7.

**Table 2. Example of a codon-optimized nucleic acid sequence encoding the fatty acid Δ11 desaturase.**

| | |
|---|---|
| **SEQ ID NO: 7** | ATGGCGCCCAACACCCGCGAGAACGAGACCATCTACGATGAGGTTGA GCATAAGCTCGAGAAACTCGTGCCTCCTCAAGCGGCCCCTGGAACT ACAAAATCGTTTATCTCAACCTTCTCACCTTCTCTTACTGGCTTATC GCCGGCGCCTACGGTCTCTATCTCTGTTTACCTCCGCAAAATGGGC CACCATCATCTTCGAGTTCATCCTCTTCTTTTCGCCGAGATGGGCA TCACCGCAGGTGCTCACCGCCTCTGGACCCATAAATCTTACAAAGCC AAGCTCCCTCTCGAGATCTTCCTCATGGTGCTCAATTCTGTTGCGTT CCAAAACACGGCCACGGACTGGGTGCGCGATCATCGCCTCCACCATA AGTACTCCGACACCGACGCGGATCCTCACAACGCTGCGCGGTCTC TTCTTCTCCCATGTCGGTTGGCTCCTCGTCCGCAAGCACGACGAGGT CAAGAAGCGCGGTAAGTTTACCGATATGTCCGATATCTACAACAATC CCGTGCTCAAGTTCCAGAAGAAATATGCCATCCCCTTCATCGGTGCC GTTTGCTTTATCTTACCTACCGTGATCCCCATGTACTTTTGGGGTGA GTCCCTCAACAACGCCTGGCACATCTGTATCCTCCGCTATGCGATGA ACCTCAACGTCACCTTCTCCGTGAACTCCCTCGCGCATATCTGGGGT AATAAGCCCTACGACAAGGATATCAAACCCGCTCAGAACTTCGGTGT TACCCTCGCGACCTTCGGTGAGGGTTTTCACAACTATCACCACGTGT TCCCCTGGGACTATCGCACCTCCGAGCTCGGCGACAACAAGTTCAAT TTCACCACCAAATTCATCAATTTCTTTGAGCGCATCGGTCTCGCGTA TGATCTCAAGACCGTTTCCGATGACGTTATCGCGCAACGCGCCAAAC GCACCGGTGATGGTACCCATCTCTGGGATTGCGCCGATAAGAATAAT AACGATGTTGTTCAAACCAAAGCGCAAATCGATACCCTCTGCACCAA ACATGAGTACCCCTACGACGTGCCCGACTACGCCTAACATATGCCAT GGTGTCAAAACCGGGGTTAGTGACATTGACTTGTTGACAAAAATCTG TATAGCTAGAAAACTCTAAGCAACGCTTTTCTTTGTTTTATTTTTTA TGTTTAAACTCCTTCAGAATTGTAGGATATCTTGTTTTGAAAAATCC AGGACTGAGTTTCGTTGCCCCATTTGCTTGTTCTCGTTTGAAATGTC GAACAATAGAAATGCTTGCAGAATGA |

The nucleic acid sequence SEQ ID NO: 7 has been codon-optimized to encode the amino acid sequence SEQ ID NO: 1 in *Aurantiochytrium limacinum.*

For example, a sequence encoding a fatty acid Δ11 desaturase from an insect can be codon optimized to be expressed in *Aurantiochytrium* using the codon usage table shown in Table 3.

**Table 3. Codon usage table for heterologous expression in Aurantiochytrium (based on 30192 residues of A. limacinum).**

| Codon | Aminoacid | *A. limacinum* Percentage |
|---|---|---|
| TAA | * (stop) | 52.50% |
| TAG | * (stop | 20.20% |
| TGA | * (stop) | 27.40% |
| GCA | A | 31.40% |
| GCC | A | 21.20% |
| GCG | A | 14.40% |
| GCT | A | 33.00% |
| TGC | C | 55.40% |
| TGT | C | 44.60% |
| GAC | D | 43.20% |
| GAT | D | 56.80% |
| GAA | E | 47.70% |
| GAG | E | 52.30% |
| TTC | F | 41.10% |
| TIT | F | 58.90% |
| GGA | G | 25.70% |
| GGC | G | 30.30% |
| GGG | G | 11.70% |
| GGT | G | 32.40% |
| CAC | H | 47.70% |
| CAT | H | 52.30% |
| ATA | I | 11.20% |
| ATC | I | 35.60% |
| ATT | I | 53.20% |
| AAA | K | 42.30% |
| AAG | K | 57.70% |
| CTA | L | 8.60% |
| CTC | L | 21.70% |
| CTG | L | 13.10% |
| CTT | L | 30.40% |
| TTA | L | 9.30% |
| TTG | L | 16.90% |
| ATG | M | 100.00% |
| AAC | N | 51.70% |
| AAT | N | 48.30% |
| CCA | P | 28.80% |
| CCC | P | 18.90% |
| CCG | P | 17.10% |
| CCT | P | 35.30% |
| CAA | Q | 51.70% |
| CAG | Q | 48.30% |
| AGA | R | 11.90% |
| AGG | R | 8.50% |
| CGA | R | 16.20% |
| CGC | R | 27.50% |
| CGG | R | 9.40% |
| CGT | R | 26.60% |
| AGC | S | 17.50% |
| AGT | S | 15.00% |
| TCA | S | 18.50% |
| TCC | S | 13.70% |
| TCG | S | 11.80% |
| TCT | S | 23.60% |
| ACA | T | 30.40% |
| ACC | T | 23.80% |
| ACG | T | 15.90% |
| ACT | T | 29.90% |
| GTA | V | 18.50% |
| GTC | V | 22.30% |
| GTG | V | 26.50% |
| GTT | V | 32.60% |
| TGG | W | 100.00% |
| TAC | Y | 52.20% |
| TAT | Y | 47.80% |

Using an appropriate codon usage table, the expression of an exogenous enzyme in a given microorganism (such as a protist) can be boosted by replacing the original codons by the codons which are the most frequently used by said protist.

In another aspect, the invention relates to an expression cassette comprising a nucleic acid encoding a fatty acid Δ11 desaturase as defined above under the control of a promoter which is functional in a protist.

In an embodiment, the invention relates to an expression cassette comprising a nucleic acid encoding a fatty acid Δ11 desaturase as defined above under the control of a promoter which is functional in a microalgae.

In an embodiment, the invention relates to an expression cassette comprising a nucleic acid encoding a fatty acid Δ11 desaturase as defined above under the control of a promoter which is functional in a protist which is selected from the phylogenetic group SAR.

In an embodiment, the invention relates to an expression cassette comprising a nucleic acid encoding a fatty acid Δ11 desaturase as defined above under the control of a promoter which is functional in a protist which is selected from the supergroup Chromalveolata.

In an embodiment, the invention relates to an expression cassette comprising a nucleic acid encoding a fatty acid Δ11 desaturase as defined above under the control of a promoter which is functional in a Traustochytrid, preferably from a genus selected from the group consisting of *Aurantiochytrium, Japonochytrium, Sicyoidochytrium, Ulkenia, Parietichytrium, Botryochytrium, Schizochytrium, Monorhizochytrium* and *Thraustochytrium.* more preferably from the species *Aurantiochytrium limacinum* and *Aurantiochytrium mangrovei.*

In an embodiment, the invention relates to an expression cassette as defined above which comprises or consists of the sequence SEQ ID NO: 8.

**Table 4. Example of a recombinant cassette for the expression of the fatty acid Δ11 desaturase in Aurantiochytrium limacinum.**

| | |
|---|---|
| **SEQ ID NO: 8** | CTGCAGGTAGGTAGGTGGCAGTAGCGTTACGAGGAGGAGTCCCGAGA GGGAGTCGGAGAGTAGAAAACTGGAAGTCGGCGAAACAAAAGGCGCA GAGATTTGCCGGAATGGAGAGTTATCGTGAGACTCTCTGAGTAGACC CAAGTGTCCTGTGAGGCACTCGTGATAGGGAGGGGGCACGGCTGAA GGGGGCTACAGTAAGGAGAGAGTGGCGTCAGTGGGGTTTTGCCGAGA ACTCTTCGAGAAAGAGGAAGAGAGGAACCGAGAGCGCCGTTGAAGAG GGGAAAAAGCAGACGGTTTAATTATAATTAATTAAGTAATTAATTAC TTACTTATTGATTGATTGATTTGAGAAGAGAAGCAAAGAGAGAGTTG AAGAAATAGTAACGAAGAATAGGAGAAGAAAGGGGCAAGAAAAGAAA AAGAAAGAGGAGAATATTAGTCGATGAGCGAGAACGTGCAAATCCAA AACAGCAAAACTCAAACTCAAACTCAAACTACAAGAAGCGTGGCGTT GCAGAGGCAACAGCTCGAAAGCAACACAGAACAAACAAACACAGGAG AGGCAGTAAGGTCAATTTCGCGGCCGCGCTAGCATGGCGCCCAACAC CCGCGAGAACGAGACCATCTACGATGAGGTTGAGCATAAGCTCGAGA AACTCGTGCCTCCTCAAGCGGGCCCCTGGAACTACAAAATCGTTTAT CTCAACCTTCTCACCTTCTCTTACTGGCTTATCGCCGGCGCCTACGG TCTCTATCTCTGTTTTACCTCCGCAAAATGGGCCACCATCATCTTCG AGTTCATCCTCTTCTTTTTCGCCGAGATGGGCATCACCGCAGGTGCT CACCGCCTCTGGACCCATAAATCTTACAAAGCCAAGCTCCCTCTCGA GATCTTCCTCATGGTGCTCAATTCTGTTGCGTTCCAAAACACGGCCA CGGACTGGGTGCGCGATCATCGCCTCCACCATAAGTACTCCGACACC GACGCGGATCCTCACAACGCTGCGCGCGGTCTCTTCTTCTCCCATGT CGGTTGGCTCCTCGTCCGCAAGCACGACGAGGTCAAGAAGCGCGGTA AGTTTACCGATATGTCCGATATCTACAACAATCCCGTGCTCAAGTTC CAGAAGAAATATGCCATCCCCTTCATCGGTGCCGTTTGCTTTATCTT ACCTACCGTGATCCCCATGTACTTTTGGGGTGAGTCCCTCAACAACG CCTGGCACATCTGTATCCTCCGCTATGCGATGAACCTCAACGTCACC TTCTCCGTGAACTCCCTCGCGCATATCTGGGGTAATAAGCCCTACGA CAAGGATATCAAACCCGCTCAGAACTTCGGTGTTACCCTCGCGACCT |
| | TCGGTGAGGGTTTTCACAACTATCACCACGTGTTCCCCTGGACTAT CGCACCTCCGAGCTCGGCGACAACAAGTTCAATTTCACCACCAAATT CATCAATTTCTTTGAGCGCATCGGTCTCGCGTATGATCTCAAGACCG TTTCCGATGACGTTATCGCGCAACGCGCCAAACGCACCGGTGATGGT ACCCATCTCTGGGATTGCGCCGATAAGAATAATAACGATGTTGTTCA AACCAAAGCGCAAATCGATACCCTCTGCACCAAACATGAGTACCCCT ACGACGTGCCCGACTACGCCTAACATATGCCATGGTGTCAAAACCGG GGTTAGTGACATTGACTTGTTGACAAAAATCTGTATAGCTAGAAAAC TCTAAGCAACGCTTTTCTTTGTTTTATTTTTTATGTTTAAACTCCTT CAGAATTGTAGGATATCTTGTTTTGAAAAATCCAGGACTGAGTTTCG TTGCCCCATTTGCTTGTTCTCGTTTGAAATGTCGAACAATAGAAATG CTTGCAGAATGAGGTTCTCCTTTACAAAAAAACTCGATAGGGTTCAA TATGAAGCTGTCTCAATGCATAGATTTCCACGATTTTACCTTTGCAT AATCTATGGTGCGCGTCAGATGCCACCCTCGTCGCTGTACAACCAAT ACATTGTAGCTTCATTTTGACATTAGGTACCTTCTTCCCCGACCTCC TTCAGAATCTCAGAGTAAGCGATCGTCACCCCTTCTACCTGAAACTC TACCACTGCATACGTAGTAAAGGCCTCTAATTACCACGGTAGTACTA TTCTTGCACTGAGGAATTCTCTAGACGAATGTAGGCTATTCTTAATG GACCGGCCCTCAGCTCGATTATTTTTGCTTGACTTGACTTGACTTGA TTCATGAAGTTGATAGGAAAGAAACATAACCCATCCCATCCCACAAC CTGCGTGTACTCTGATCGGCAGGTGCACGCTGAGTTGAAGGTGGTTC AAGAATCGAAAACATCAGCCTAGAGCACGACGAGGTTTCAGAGAGCC AACTTTTTCTATCTATTAATCTCATCCTTTGCTTCTTCGCGGACAAC GACGGTGGATCAGCGCCGCCGCTGAGAAGACAGCAGAGGTAACTCTA GCAAGAGAAGCAGCAGTAGCTTCGTCTGGTCAAGAGACTCTGCTTAA GCACAGTAGCCTGCAAATAAAGACACTTGGGCAAAAGAAACATTGAC ATTGATTGAATTTCACGCAGAGGCAAATGGAAGCTT |

The nucleic acid sequence SEQ ID NO: 8 contains the nucleic acid sequence SEQ ID NO: 7 (shown in bold in Table 3) and allows the expression of the desaturase of amino acid sequence SEQ ID NO: 1 in *Aurantiochytrium limacinum.*

In another aspect, the invention relates to a vector comprising a nucleic acid as defined above or an expression cassette as defined above.

As used herein, a "vector" is a nucleic acid molecule used as a vehicle to transfer genetic material into a cell. The term "vector" encompasses plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences.

In another aspect, the invention relates to a protist comprising:
- a nucleic acid as defined above,
- an expression cassette as defined above, or
- a vector as defined above.

A protist expressing the recombinant enzyme can be referred to as a "modified", "transgenic" or "transformed" protist.

The invention furthermore relates to the use of a protist as defined above as feeds (for fisheries), as food supplements, cosmetic supplements or health supplements, for the production of polymers in green industry or for the production of biofuels.

The following figures and examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1****.** Schematic representation of the plasmid pUbi-d11Tp encoding the Δ11 desaturase from T. *pityocampa.*
**Figure 2****.** Schematic representation of the plasmid pUbi-Zeo encoding the zeocin resistance.
**Figure 3****.** Schematic representation of the plasmid pUbi-d11Tp encoding the Δ11 desaturase from T. *pseudonana.*
**Figure 4****.** Dot plot of the sequence identity values calculated on a multialignment containing 484 delta11 desaturase sequences from the class Insecta. In x-axis: sequences, in *y*-axis: sequence identity value.
**Figure 5****.** Neighbor-Joining phylogenetic tree constructed with a subset of Δ11 sequences retrieved from NCBI. In the figure, the orders within the class Insecta of which the sequences belong are reported. For Lepidoptera, two groups have been identified: the butterflies and the moths. A black star identifies the *Thaumetopoea pityocampa* acyl-CoA Δ11 desaturase sequence.
**Figure 6****.** Fresh weight (A) and optical density at 600nm (B) of the transgenic lines and control cultures after 5 days of culture run in parallel.
**Figure 7****.** Fatty Acid content in the transgenic lines and control cultures at days 2 and 5. The bold lines show the upper and lower range values for controls at day 5. Inset: picture of the chloroform extracted lipids in one of the transgenic lines (tube on the right) and a control (tube on the left). Note the different color intensities indicating a much higher oil content in the transgenic line.
**Figure 8****.** TAG content (A) and polar lipids (B) in control (empty vector) and four mutants overexpressing the Acyl-CoA Δ11 desaturase from the moth *Thaumetopoea pityocampa.*
**Figure 9****.** Fatty Acid composition (%) in transgenic lines and controls after 5 days of culture.
**Figure 10****.** DHA (22:6) and DPA (22:5) content in transgenic (dark grey) and control (light grey) cell lines.
**Figure 11****.** Dry weight of the transgenic lines and control cultures after 2 and 5 days of culture run in parallel.
**Figure 12****.** Fatty Acid content in the transgenic lines and control cultures at days 2 and 5.
**Figure 13****.** DPA (22:5) and DHA (22:6) content in transgenic (dark grey) and control (light grey) cell lines.

### EXAMPLES

### Materials and Methods

### Cultivation and transformation of Aurantiochytrium

The thraustochytrid used in the examples is an *Aurantiochytrium* species (*Aurantiochytrium limacinum*). It was cultivated in R medium containing the ingredients listed in Table 5:

**Table 5. Composition of the R medium.**

| Component | Final Concentration (w/v) |
|---|---|
| NaCl | 10.597 g/l |
| Na₂SO₄ | 1.775 g/l |
| NaHCO₃ | 87 mg/l |
| KCl | 299.5 mg/l |
| KBr | 43.15 mg/l |
| H₃BO₃ | 11.5 mg/l |
| NaF | 1.4 mg/l |
| MgCl₂•6H₂O | 4.796 g/l |
| CaCl₂•2H₂O | 0.672 g/l |
| SrCl₂•6H₂O | 10.9 mg/l |
| EDTA-iron | 1.50 mg/l |
| Na₂EDTA•2H₂O | 1.545 mg/l |
| ZnSO₄•7H₂O | 36.5 µg/l |
| CoCl₂•6H₂O | 8 µg/l |
| MnCl₂•4H₂O | 0.27 mg/l |
| Na₂MoO₄•2H₂O | 0.74 µg/l |
| Na₂SeO₃ | 0.085 µg/l |
| NiCl₂•6H₂O | 0.745 µg/l |
| CuSO₄•5H₂O | 4.9 µg/l |
| Vitamin H | 0.499 µg/l |
| Vitamin B12 | 0.501 µg/l |
| Vitamin B1 | 78.54 µg/l |
| NaNO₃ | 23.33 mg/l |
| NaH₂PO₄ | 1.343 mg/l |
| Glucose | 60 g/l |
| Yeast extract | 20 g/l |

50 ml cultures were grown in sterile 250ml Pyrex flasks under agitation (100 rpm). Solid medium has the same composition as Table 5 but contains 1% agar.

### Cassette for the expression of Acyl-CoA Δ11 desaturase from Thaumetopoea pityocampa

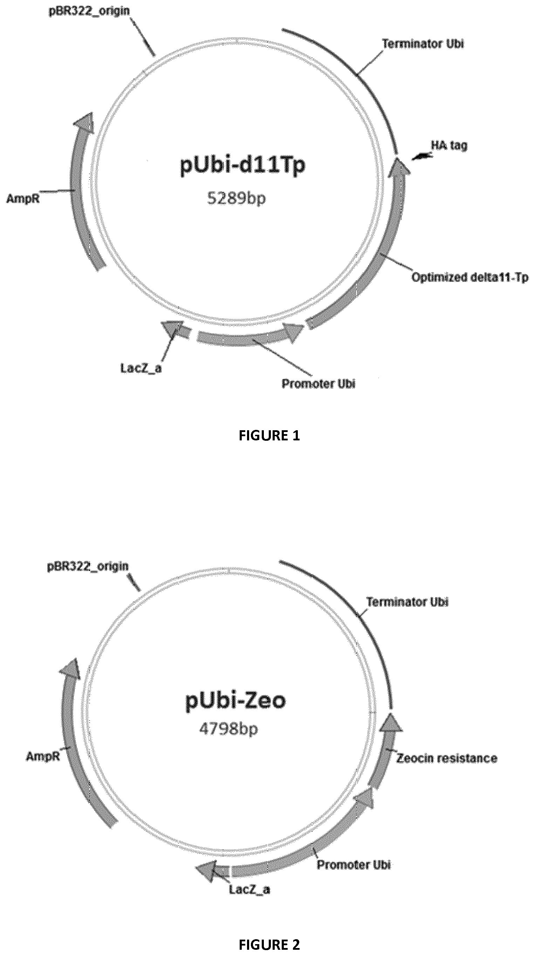

The polynucleotide coding for an acyl-CoA Δ11 desaturase from the moth *Thaumetopoea pityocampa* (SEQ ID NO: 1) was codon optimized using a homemade codon usage table (based on 30192 residues, see also Table 3) for heterologous over-expression in *Aurantiochytrium* under the control of the polyubiquitin endogenous gene promoter. The transcription terminator used in this construct was the endogenous polyubiquitin gene terminator. An HA tag sequence (YPYDVPDYA, SEQ ID NO: 9) was added between the last encoding amino acid and the stop codon of the acyl-CoA Δ11 desaturase sequence. Two restriction sites were added at the 5' end (NotI, NheI) and the 3' end (Ndel, Ncol) of the DNA sequence, producing the optimized delta11Tp-HA gene. The final delta11-Tp cassette (SEQ ID NO: 8), containing the Ubi promoter region from the pUbi-Zeo, followed by the optimized delta11Tp-HA gene, and the Ubi terminator region from the pUbi-Zeo, was synthesized and subcloned into a commercial pUC19 plasmid using Pstl/HindIII restriction sites by the Invitrogen GeneArt Gene Synthesis Service to obtain the vector pUbi-d11Tp (Figure 1). The plasmid was co-transformed with the zeocin resistance cassette under the same polyubiquitin promoter.

### Cassette for the expression of the zeocin resistance

In order to clone the zeocin resistance gene under the polyubiquitin promoter/terminator into the expression vector, an ORF encoding a yeast UBI4 polyubiquitin homologous gene was identified in the genome of *Aurantiochytrium.* A 917 pb sequence upstream of the ORF was amplified with following primers PromUbi2Sacl-F (TTGAGCTCAGAGCGCGAAAGAGAGTGCCGGAATTC, SEQ ID NO: 10)) and PromUbi2BamHI-R (GCGGATCCGAAATTGACCTTACTGCCTCTCCTGTG, SEQ ID NO: 11) to add the restriction sites SacI in 5' and BamHI in 3' of the sequence. A 935 pb sequence downstream of the ORF was amplified with the following primers TermUbi2Sphl-F (GGGCATGCTGTCAAAACCGGGGTTAGTGACATTGA, SEQ ID NO: 12) and TermUbi2Hindlll-R (GGAAGCTTCCATTTGCCTCTGCGTGAAATTCAATC, SEQ ID NO: 13) to add the restriction sites Sphl in 5' and Hindlll in 3' of the sequence. A 375 pb sequence encoding the zeocin gene from the commercial plasmid pTEF1 was amplified with following primers ZeoS1BamHI (GCGGATCCATGGCCAAGTTGACCAGTGCCGTTCC, SEQ ID NO: 14) and ZeoS1Sall (GCGTCGACTCAGTCCTGCTCCTCGGCCACGAAGT, SEQ ID NO: 15) to add the restriction sites BamHI in 5' and Sall in 3' of the sequence. All sequences were sequentially inserted into the multiple cloning site of the pUC19 plasmid to obtain the vector pUbi-Zeo (Figure 2), using common cloning techniques (restriction enzyme digestion, ligation, *E. coli* termo-transformation, plasmid preparation). The sequence of the complete zeocin resistance cassette corresponds to SEQ ID NO: 16 (see Table 6).

**Table 6. Cassette encoding the zeocin resistance.**

| | |
|---|---|
| **SEQ ID NO: 16** Cassette encoding the zeocin resistance | GAGCTCAGAGCGCGAAAGAGAGTGCCGGATTCAAAGACGCCACAGCGGAAAG AAAGAAAGACCTAGGAGGTACTAGCTGGTTGTAGCTAGCTAGCTAGCTAGCTA GCTTATGCTGCTAAGACGCCCTTCCTCCTCGAGGTCCTTTTGACTTGCCAGCG CAGTCTCCTTTGTCTTCTTCGCTCATTTAATCAAGTCAAGTCTTCAGGTTTAA AATGAAAAATCCTGCTTCCAGGTTCAGTTCTAGCAAGTAGGTAGGTGGCAGTA GCGTTACGAGGAGGAGTCCCGAGAGGGAGTCGGAGAGTAGAAAACTGGAAGTC GGCGAAACAAAAGGCGCAGAGATTTGCCGGAATGGAGAGTTATCGTGAGACTC TCTGAGTAGACCCAAGTGTCCTGTGAGGCACTCGTGATAGGGAGGGGGCACGG GCTGAAGGGGGCTACAGTAAGGAGAGAGTGGCGTCAGTGGAGTTTCGCCGAGA ACTCTTCGAGAAAGAGGAAGAGAGGAACCGAGAGCGCCGTTGAAGAGGGGAAA AAGCAGACGGTTTAATTATAATTAATTAAGTAATTAATTACTTACTTATTGAT TGATTGATTTGAGAAGAGAAGCAAAGAGAGAGTTGAAGAAATAGTAACGAAGA ATAGGAGAAGAAAGGGGCAAGAAAAGAAAAAAGAAAGAGGAGAATATTGGTCG ATGAGCGAGAACGTGCAAATCCAAAACAGCAAAACTCAAACTCAAACTACAAG AAGCGTGGCGTTGCAGAGGCAACAGCTCGAAAGCAACACAGAACAGACAAACA CAGGAGAGGCAGTAAGGTCAATTTCGGATCCATGGCCAAGTTGACCAGTGCCG TTCCGGTGCTCACCGCGCGCGACGTCGCCGGAGCGGTCGAGTTCTGGACCGAC CGGCTCGGGTTCTCCCGGGACTTCGTGGAGGACGACTTCGCCGGTGTGGTCCG GGACGACGTGACCCTGTTCATCAGCGCGGTCCAGGACCAGGTGGTGCCGGACA ACACCCTGGCCTGGGTGTGGGTGCGCGGCCTGGACGAGCTGTACGCCGAGTGG TCGGAGGTCGTGTCCACGAACTTCCGGGACGCCTCCGGGCCGGCCATGACCGA GATCGGCGAGCAGCCGTGGGGGCAGGAGTTCGCCCTGCGCGACCCGGCCGGCA ACTGCGTGCACTTCGTGGCCGAGGAGCAGGACTGAGTCGACCTGCAGGCATGC TGTCAAAACCGGGGTTAGTGACATTGACTTGTTGACAAAAATCTGTATAGCTA GAAAACTCTAAGCAACGCTTTTCTTTGTTTTATTTTTTATGTTTTAACTCCTT CAGAATTGTAGGATATCTTGTTTTGAAAAATCCAGGACTGAGTTTCGTTGCCC CATTTGCTTGTTCTCGTTTGAAATGTCGAACAGTAGAAATGCTTGCAGAATGA GGTTCTCCTTTACAAAAACTCGATAGGGTTCAATATGAAGCTGTCTCGATGC ATAGATTTCCACGATTTTACCTTTGCATAATCTATGGTGCGCGTCAGATGCCA CCCTCGTCGCTGTACAACCAATACATTGTAGCTTCATTTTGACATTAGGTACC TTCTTCCCCGACCTCCTTCAGAATCTCAGAGTAAGCGATCGATCGTCACCCCT TCTACCTGAAACTCTACCACTGCATACGTAGTAAAGGCCTCTAATTACCACGG TAGTACTATTCTTGCACTGAGGAATTCTCTAGACGAATGTAGGCTATTCTTAA TGGACCGGCCCTCAGCTCGATTATTTTTGCTTGACTTGACTTGACTTGATTAA TGAAGTTGATAGGAAAGAAACATAACCCATTCCATCCCACAACCTGCGTGTAC TCTGATCGGCAGGTGCACGCTGAGTTGAGGGTGATTTAAGGATCGAAAACATC AGCCTAGAGCACGACGAGGTTCCAGAGAGCCAACTTTTTCTATCTATTAATCT CATCCTTTGCTTCTTCGCGGACAACGACGGTGGATCAGCGCCGCCGCTGAGAA GACAGCAGAGGTAACTCTAGCAAGAGAAGCAGCAGTAGCTTCGTCTGGTCAAG AGACTCTGCTTAAGCACAGTAGCCTGCAAATAAAGACACTTGGGCAAAAGAAA CATTGACATTGATTGAATTTCACGCAGAGGCAAATGGAAGCTT |

### Cassette for the expression of an Acyl-CoA Δ11 desaturase from Thalassiosira pseudonana

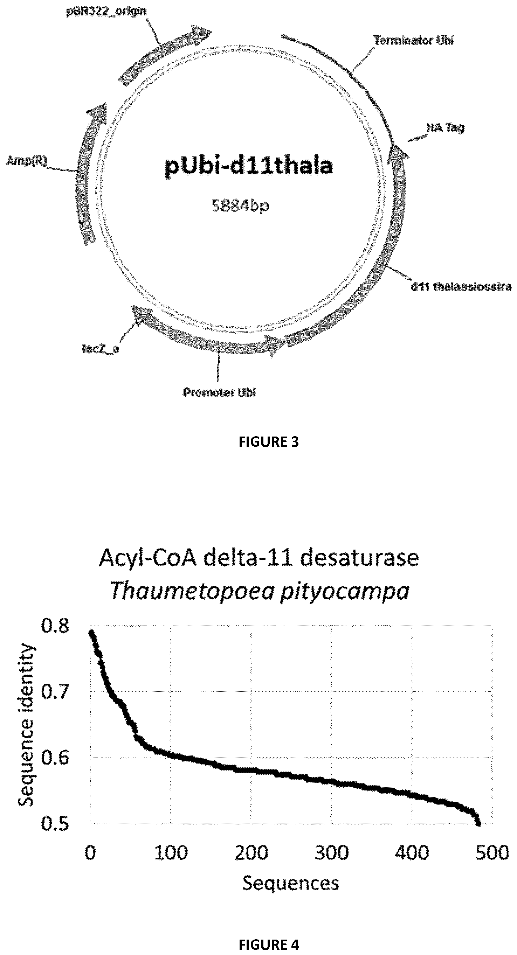

A sequence identified as a Δ11 desaturase (SEQ ID NO: 17, Thaps3| 23391, see Table 7) was found in the genome of the marine diatom *T. pseudonana.* The nucleic acid sequence encoding this Δ11 desaturase was codon-optimized and synthesized as described above. Cloning into an expression vector was performed by GeneArt© gene synthesis service to obtain pUbi-d11thala (Figure 3).

**Table 7. Codon-optimized nucleic acid sequence encoding the Acyl-CoA Δ11 desaturase from Thalassiosira pseudonana.**

| | |
|---|---|
| **SEQ ID NO: 17** Cassette encoding a Acyl-CoA Δ11 desaturase from *Thalassiosira pseudonana* | CTGCAGGTAGGTAGGTGGCAGTAGCGTTACGAGGAGGAGTCCCGAGAGGGAGT CGGAGAGTAGAAAACTGGAAGTCGGCGAAACAAAAGGCGCAGAGATTTGCCGG AATGGAGAGTTATCGTGAGACTCTCTGAGTAGACCCAAGTGTCCTGTGAGGCA CTCGTGATAGGGAGGGGGCACGGGCTGAAGGGGGCTACAGTAAGGAGAGAGTG GCGTCAGTGGGGTTTTGCCGAGAACTCTTCGAGAAAGAGGAAGAGAGGAACCG AGAGCGCCGTTGAAGAGGGGAAAAAGCAGACGGTTTAATTATAATTAATTAAG TAATTAATTACTTACTTATTGATTGATTGATTTGAGAAGAGAAGCAAAGAGAG AGTTGAAGAAATAGTAACGAAGAATAGGAGAAGAAAGGGGCAAGAAAAGAAAA AGAAAGAGGAGAATATTAGTCGATGAGCGAGAACGTGCAAATCCAAAACAGCA AAACTCAAACTCAAACTCAAACTACAAGAAGCGTGGCGTTGCAGAGGCAACAG CTCGAAAGCAACACAGAACAAACAAACACAGGAGAGGCAGTAAGGTCAATTTC GCGGCCGCGCTAGCATGGCGCCCAACACGCGGGAGAACGAGACGATCTACGAC GAAGTGGAACACAAGCTGGAGAAGCTCGTGCCCCCCCAGGCGGGCCCCTGGAA CTACAAGATCGTGTACCTGAACTTGCTGACCTTCTCCTACTGGCTGATCGCCG GCGCCTACGGTTGTACTTGTGCTTCACGTCCGCCAAGTGGGCCACGATCATC TTCGAATTCATCTTGTTCTTCTTCGCCGAGATGGGCATCACGGCCGGCGCCCA CCGGCTGTGGACGCACAAGTCCTACAAGGCCAAGTTGCCCTTGGAAATCTTCC TCATGGTGCTGAACTCCGTGGCGTTCCAGAACACGGCCACCGACTGGGTGCGG GACCACCGGCTGCACCACAAGTACAGCGACACGGACGCGGACCCCCACAACGC CGCGCGGGGGCTGTTCTTCTCCCACGTCGGGTGGCTGCTCGTCCGGAAGCACG ACGAAGTCAAGAAGCGCGGGAAGTTCACCGACATGTCCGACATCTACAACAAC CCCGTGTTGAAGTTCCAGAAGAAGTACGCCATCCCCTTCATCGGCGCCGTGTG CTTCATCTTGCCCACGGTGATCCCCATGTACTTCTGGGGCGAGTCCCTCAACA ACGCCTGGCACATCTGCATCCTGCGGTACGCGATGAACCTCAACGTCACGTTC TCCGTGAACTCCCTGGCGCACATCTGGGGCAACAAGCCCTACGACAAGGACAT CAAGCCCGCCCAGAACTTCGGCGTGACGTTGGCGACCTTCGGCGAAGGGTTCC ACAACTACCACCACGTGTTCCCCTGGGACTACCGGACGTCCGAACTCGGCGAC AACAAGTTCAACTTCACGACGAAGTTCATCAACTTCTTCGAACGGATCGGCTT GGCGTACGACCTGAAGACCGTGTCCGACGACGTGATCGCGCAGCGGGCCAAGC GGACCGGCGACGGCACGCACCTGTGGGACTGCGCCGACAAGAACAACAACGAC GTGGTGCAGACGAAGGCGCAGATCGACACCTTGTGCACGAAGCACGAATGAGG TTCTCCTTTACAAAAAAACTCGATAGGGTTCAATATGAAGCTGTCTCAATGCA TAGATTTCCACGATTTTACCTTTGCATAATCTATGGTGCGCGTCAGATGCCAC CCTCGTCGCTGTACAACCAATACATTGTAGCTTCATTTTGACATTAGGTACCT TCTTCCCCGACCTCCTTCAGAATCTCAGAGTAAGCGATCGTCACCCCTTCTAC CTGAAACTCTACCACTGCATACGTAGTAAAGGCCTCTAATTACCACGGTAGTA CTATTCTTGCACTGAGGAATTCTCTAGACGAATGTAGGCTATTCTTAATGGAC CGGCCCTCAGCTCGATTATTTTTGCTTGACTTGACTTGACTTGATTCATGAAG TTGATAGGAAAGAAACATAACCCATCCCATCCCACAACCTGCGTGTACTCTGA TCGGCAGGTGCACGCTGAGTTGAAGGTGGTTCAAGAATCGAAAACATCAGCCT AGAGCACGACGAGGTTTCAGAGAGCCAACTTTTTCTATCTATTAATCTCATCC TTTGCTTCTTCGCGGACAACGACGGTGGATCAGCGCCGCCGCTGAGAAGACAG CAGAGGTAACTCTAGCAAGAGAAGCAGCAGTAGCTTCGTCTGGTCAAGAGACT CTGCTTAAGCACAGTAGCCTGCAAATAAAGACACTTGGGCAAAAGAAACATTG ACATTGATTGAATTTCACGCAGAGGCAAATGGAAGCTT |

### Genetic transformation

Genetic transformation was performed by biolistic method. 2x107 cells of Aurantiochytrium, from a 2 to 4-day old-culture, were plated onto solid medium with 200 µg/ml zeocin in 10 cm Petri dishes. Cells were left air-dry in a sterile hood. One to two µg of each plasmid for co-transformation were coated on 25 µl of 0.7 µm diameter tungsten microcarriers (hereon referred to as 'beads'). 25 µL of CaCl2 2.5 M in absolute ethanol and 10 µL spermidine were added to the beads then 4 volumes of absolute ethanol to wash the beads. The beads were spun down for 6-7 sec at 8000 g, the supernatant discarded and 700 µl ice cold ethanol was added again. The supernatant was discarded and the pellet suspended in 25 µl ethanol. Coated beads were kept on ice until use. The particle bombardment was performed with a PDS-1000/He Particle Delivery System equipped with a rupture disk resistance 1550 psi. 10µl of the bead mix was placed on the macrocarriers. Two shots per bead preparation were performed.

Genetic transformation of *Aurantiochytrium* can be achieved by other methods, such as electroporation.

### Lipid extraction and fatty acid analyses

Glycerolipids were extracted from freeze-dried cells. First, cells were harvested by centrifugation and snap-frozen in liquid nitrogen. Ten mg dry weight were suspended in 4 mL of boiling ethanol for 5 minutes. Lipids were extracted by addition of 2 mL methanol and 8 mL chloroform at room temperature (as described in Folch T et al., Journal of Biological Chemistry, 226:497-509, 1957). The mixture was saturated with argon and stirred for 1 hour at room temperature. After filtration through glass wool, cell remains were rinsed with 3 mL chloroform/methanol 2:1, v/v. Five mL of NaCl 1% were added to the filtrate to initiate biphase formation. The chloroform phase was dried under argon before solubilizing the lipid extract in pure chloroform (as described in Jouhet J et al., PLoS One, 12(8):e0182423, 2017).

Total fatty acids were analyzed as follows: in an aliquot fraction, a known quantity of 21:0 was added and the fatty acids present were converted to methyl esters (fatty acid methyl ester or FAME) by a 1-hour incubation in 3 mL 2.5% H2SO4 in pure methanol at 100°C (as described in Jouhet et al., FEBS Letters, 544(1-3):63-8, 2003). The reaction was stopped by adding 3 mL 1:1 water:hexane. The hexane phase was analyzed by gas chromatography (gas chromatography coupled to mass spectrometry and flame ionization detection, GC-MS/FID) (Perkin Elmer, Clarus SQ 8 GC/MS series) on a BPX70 (SGE) column. FAMEs were identified by comparison of their retention times with those of standards (obtained from Sigma) and quantified using 21:0 for calibration. Extraction and quantification were performed at least 3 times.

### Quantification of glycerolipids by high performance liquid chromatography (HPLC) and tandem mass spectrometry (MS/MS) analyses

The various glycerolipids were routinely quantified using an external standard corresponding to a qualified control (QC) of lipids extracted from the same strain (as described in Jouhet J et al., PLoS One, 12(8):e0182423, 2017). This QC extract was a known lipid extract previously qualified and quantified by thin layer chromatography (TLC) and GC-MS/FID, as described above. For the routine analyses of the samples, lipids corresponding to 25 nmol of total fatty acids were dissolved in 100 µL of chloroform/methanol [2/1, (v/v)] containing 125 pmol each of DAG 18:0-22:6, PE 18:0-18:0 and SQDG 16:0-18:0 as internal standard (Avanti Polar Lipids Inc). All the internal standard solutions were first quantified by GC-FID. Lipids were then separated by HPLC and identified by ESI-MS/MS.

The HPLC separation method was adapted from Rainteau et al. (PLoS One, 7(7):e4198510, 2012). Lipid classes were separated using an Agilent 1200 HPLC system using a 150 mm x 3 mm (length x internal diameter) 5 µm diol column (Macherey-Nagel), at 40°C. The mobile phases consisted of hexane/isopropanol/water/ammonium acetate 1M, pH5.3 [625/350/24/1, (v/v/v/v)] (A) and isopropanol/water/ammonium acetate 1M, pH5.3 [850/149/1, (v/v/v)] (B). The injection volume was 20 µL. After 5 min, the percentage of B was increased linearly from 0% to 100% in 30 min and stayed at 100% for 15 min. This elution sequence was followed by a return to 100% A in 5 min and an equilibration for 20 min with 100% A before the next injection, leading to a total runtime of 70 min. The flow rate of the mobile phase was 200 µL/min. The distinct glycerophospholipid classes were eluted successively as a function of the polar head group. Under these conditions, they were eluted in the following order: Triacylglycerols (TAG), Diacylglycerols (DAG), Phosphatidylethanolamines (PE), Phosphatidylglyecrols (PG), Phosphatidylinositols (PI), Phosphatidylserines (PS), Phosphatidylcholines (PC), Diphosphatidylglycerols (DPG) and Phosphatidic acids (PA).

Mass spectrometric analysis was done on a 6460 triple quadrupole mass spectrometer (Agilent) equipped with a Jet stream electrospray ion source under following settings: Drying gas heater: 260°C, Drying gas flow 13 L/min, Sheath gas heater: 300°C, Sheath gas flow: 11L/min, Nebulizer pressure: 25 psi, Capillary voltage: ± 5000 V, Nozzle voltage ± 1000. Nitrogen was used as collision gas. The quadrupoles Q1 and Q3 were operated at widest and unit resolution respectively. Mass spectra were processed by MassHunter Workstation software (Agilent). The QC sample is used as an external standard, and run with the list of the samples to be analyzed. First, lipid amounts in all samples were adjusted with three internal standards (see above) to correct possible variations linked to the injection and analytical run. Then, within the QC samples, molecules in a given class of glycerolipid were summed and compared to the amount of the same lipid class previously determined by TLC-GC. This is done in order to establish a correspondence between the area of the peaks and a number of pmoles. These corresponding factors were then applied to the samples of the list to be analyzed.

### Example I. Conservation of the Acyl-CoA Δ11 desaturase among insects

A multialignment was carried out on 484 Δ11 sequences retrieved from the NCBI database using *Thaumetopoea pityocampa* acyl-CoA Δ11 desaturase as query (SEQ ID NO: 1). The sequences in fasta format were imported in BioEdit computer program and aligned using the ClustalW algorithm implemented in BioEdit. The alignment was trimmed in N-ter and C-ter taking into account the functional domains of the proteins. A sequence identity matrix was produced using the utility implemented in BioEdit software and the sequence identity values of *Thaumetopoea pityocampa* acyl-CoA Δ11 desaturase vs all the other 483 sequences in the alignment was plotted in Figure 4. The 100% identity (value 1.00) of the comparison with the *Thaumetopoea pityocampa* acyl-CoA Δ11 desaturase sequence itself was not included in the dot plot. 23% (114) of the sequences presented an identity above 60%, 22% (107) an identity below 55%. All the amino acid sequences analyzed have a % identity equal or above 50% compared to SEQ ID NO: 1.

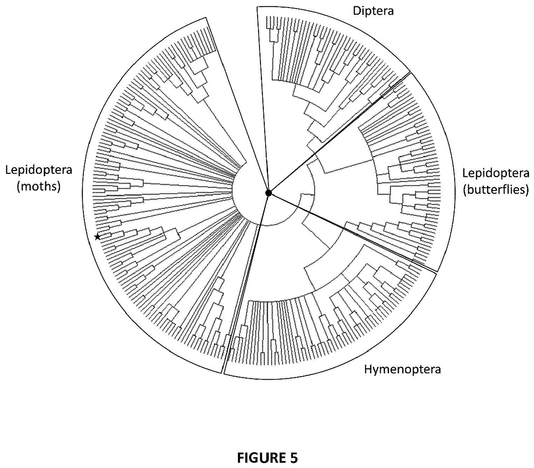

A subset of the alignment produced as described above, was used to construct a phylogenetic tree (Figure 5). The evolutionary history was inferred using the Neighbor-Joining method. The bootstrap consensus tree inferred from 1000 replicates is taken to represent the evolutionary history of the taxa analyzed. Branches corresponding to partitions reproduced in less than 50% bootstrap replicates are collapsed. The evolutionary distances were computed using the Poisson correction method and are in the units of the number of amino acid substitutions per site. The rate variation among sites was modeled with a gamma distribution (shape parameter = 1). The analysis involved 285 amino acid sequences. All ambiguous positions were removed for each sequence pair. There were a total of 300 positions in the final dataset. Evolutionary analyses were conducted in MEGA7.

### Example II. Production of fatty acids by Aurantiochytrium clones expressing the Acyl-CoA Δ11 desaturase from Thaumetopoea pityocampa

Four transformant *Aurantiochytrium* clones, Thom7, Thom8, Thom10, Thom23', obtained after transformation with the vector pUbi-d11Tp were PCR validated for the presence of the transgene in the genome and then used for the determination of their growth rate and lipid content. A wild type culture and a transformation control (pUbiZeo5) were added. The latter clone was transformed with the zeocin resistance cassette only and is meant to give the lipid baseline production for a biolistics-derived transformant. Growth was followed by measuring the fresh weight and the optical density at 600 nm of the cultures over a period of 5 days. Lipid measurements were performed on days 2 and 5. All the experiments were run in biological independent duplicates, except for pUbiZeo5 where two independent experiments were run, each in duplicate.

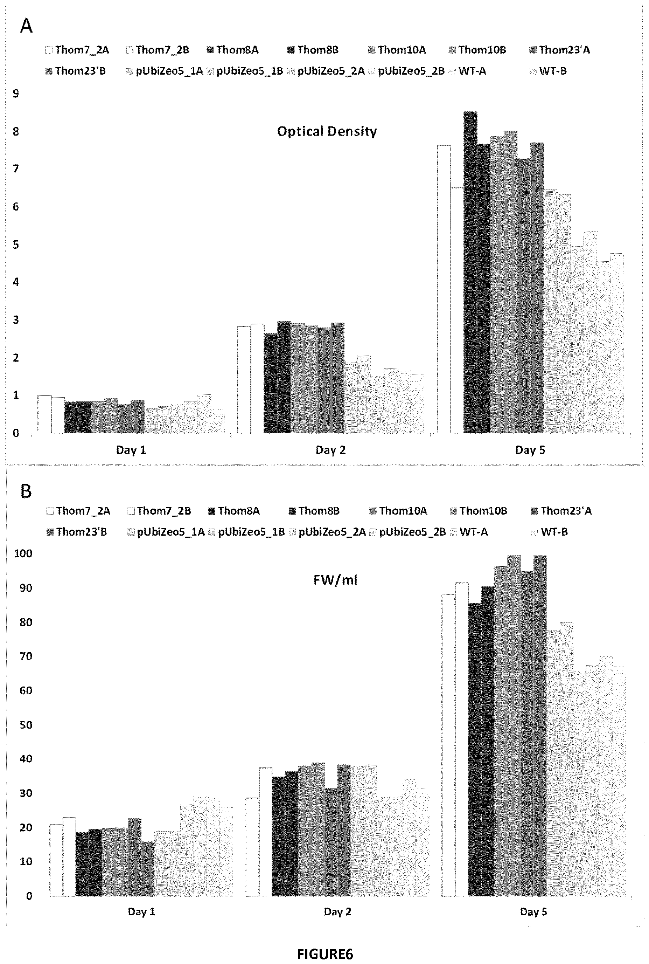

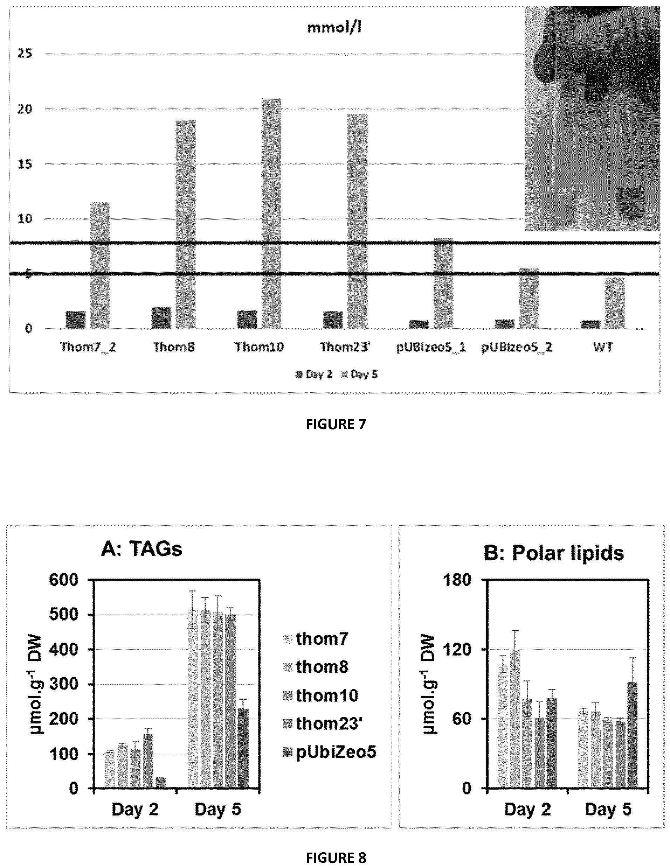

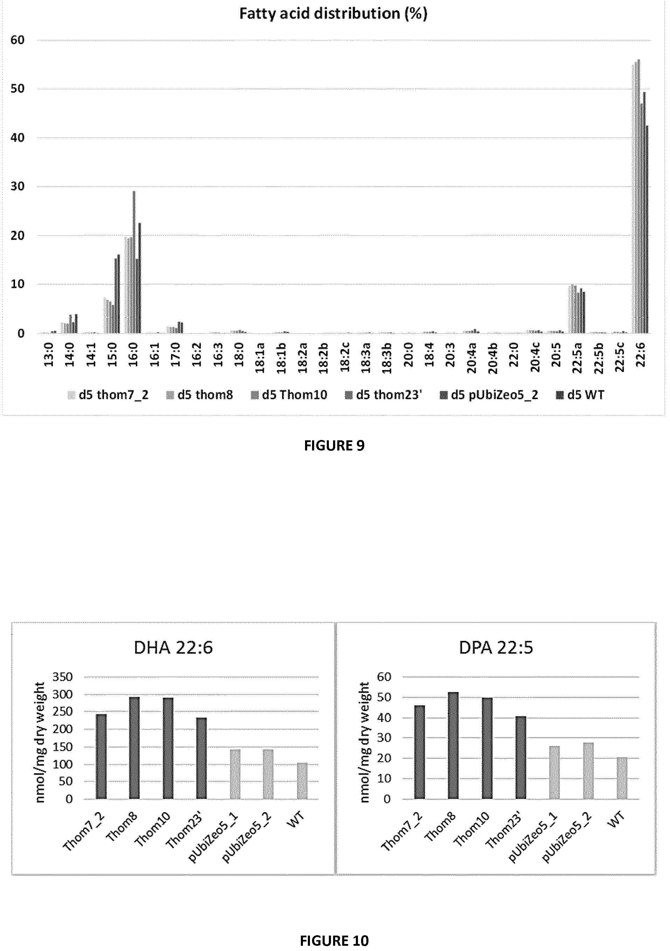

Fresh weight was comparable among transformants and controls during the first two days of the experiment, but at day 5 the four transformants had accumulated more biomass (higher fresh weight per milliliter of culture) and displayed a higher optical density (Figure 6A and 6B) than the controls. In addition, the four screened clones produced on average 2 times more total fatty acids per ml of culture (Figure 7) than the controls on day 2 and 2-3 times more on day 5. The TAG content expressed as µmol per mg of fresh weight also increased by a factor of 2 (Figure 8A), whereas the polar (membrane) lipid content was little affected (Figure 8B), indicating that the increase of fatty acids was due to a higher level of lipid storage (TAGs). The FAME profiles of the transformants displayed a lower proportion of 15:0 compared to controls, and three transformants (Thom7, Thom8, Thom10) out of the four showed an augmented percentage of DHA. On average at day 5 the transgenic lines presented >54% DHA while the controls showed 45% (Figure 9). In the transgenic lines Thom7, Thom8, Thom10, Thom23', the PUFA content (DHA, 22:6 and DPA, 22:5) expressed per mg dry weight was on average twice as much as the controls at day 5 (Figure 10) and, taking into account that they also produced more biomass (Figure 6A), the yield of lipid production (µmoles / ml culture) was about three times higher.

### Example III. Production of fatty acids by Aurantiochytrium clones expressing the Acyl-CoA Δ11 desaturase from Thalassiosira pseudonana (comparative example)

In WO 2005/080578, desaturases from the diatom *Thalassiosira pseudonana* were identified (TpDESN) and functionally characterized in *T. pseudonana* and in yeast. By supplementing the culture media with different fatty acids, it was possible to identify such a Δ11 desaturase as not being a front-end desaturase albeit its primary sequence shows high similarity with this protein family. TpDESN acts primarily on 16:0. The expression of this protein in the yeast led to the production of specific fatty acids upon culture medium supplementation with different fatty acid substrates.

A sequence identified as a Δ11 desaturase (SEQ ID NO: 17, Thaps3 | 23391) was found in the genome of the marine diatom *T. pseudonana. Aurantiochytrium* was transformed to express this Δ11 desaturase. This Δ11 desaturase showed 10.6% homology with the *Thaumetopoea pityocampa* acyl-CoA Δ11 desaturase of Example 2.

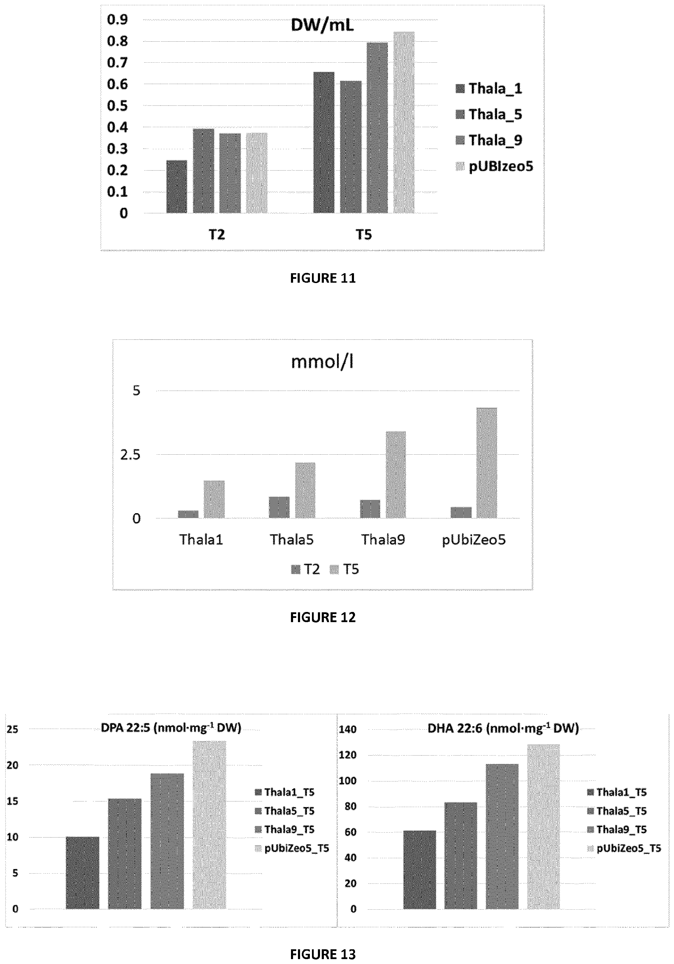

Three transformant *Aurantiochytrium* clones were analyzed, Thala1, Thala5, and Thala9. Growth and biomass accumulation was slightly affected in all the transformants compared to the pUbiZeo5 negative control (Figure 11).

The total fatty acid production was affected in transformant clones (Figure 12) as well as the DHA and DPA content (Figure 13), showing reduced fatty acid and PUFA contents.

## Claims

1. The use of a recombinant Δ11 fatty acid desaturase comprising or consisting of a sequence having at least 50% identity with the sequence SEQ ID NO: 1 for increasing the content of triacylglycerides and/or the content of fatty acids in a protist.

2. A method for producing triacylglycerides and/or fatty acids, wherein said method comprises a step of expression of a recombinant fatty acid Δ11 desaturase comprising or consisting of a sequence having at least 50% identity with the sequence SEQ ID NO: 1 in a protist.

3. The use according to claim 1, or the method according to claim 2, wherein said protist is a microalgae.

4. The use according to claims 1 or 3, or the method according to claims 2 or 3, wherein said protist is a Thraustochytrid, preferably from a genus selected from the group consisting of *Aurantiochytrium, Japonochytrium, Sicyoidochytrium, Ulkenia, Parietichytrium, Botryochytrium, Schizochytrium, Monorhizochytrium* and *Thraustochytrium,* more preferably selected from the species *Aurantiochytrium limacinum* and *Aurantiochytrium mangrovei.*

5. The use according to claims 1 or 3-4, or the method according to claims 2-4, wherein said fatty acids are polyunsaturated fatty acids, preferably long-chain polyunsaturated fatty acids or very long-chain polyunsaturated fatty acids.

6. The use according to claims 1 or 3-5, or the method according to claims 2-5, wherein said fatty acids are eicosapentaenoic acid (EPA, 20:5), docosapentaenoic acid (DPA, 22:5) or docosahexaenoic acid (DHA, 22:6).

7. The use according to claims 1 or 3-6, or the method according to claims 2-6, wherein said recombinant Acyl-CoA Δ11 desaturase comprises or consists of the sequence SEQ ID NO: 1, SEQ ID NO: 2 or SEQ ID NO: 3.

8. A nucleic acid encoding a fatty acid Δ11 desaturase comprising or consisting of a sequence having at least 50% identity with the sequence SEQ ID NO: 1, said nucleic acid being codon-optimized for the expression of said fatty acid Δ11 desaturase in a protist.

9. The nucleic acid according to claim 8 which comprises or consists of the sequence SEQ ID NO: 7.

10. An expression cassette comprising a nucleic acid encoding a fatty acid Δ11 desaturase as defined in claims 8 or 9, said nucleic acid encoding a fatty acid Δ11 desaturase being under the control of a promoter which is functional in a protist.

11. A vector comprising a nucleic acid as defined in claims 8 or 9 or an expression cassette as defined in claim 10.

12. A protist comprising:
- a nucleic acid as defined in claim 8 or 9,
- an expression cassette as defined in claim 10, or
- a vector as defined in claim 11.
